# EUROPEAN PATENT APPLICATION

(11) **EP 4 273 233 A2**
(43) Date of publication of application: **08.11.2023**
(21) Application number: 23181086.2
(22) Date of filing: 07.03.2018
(51) Int. Cl.: C12N 5/0783

(54) **MR1 RESTRICTED T CELL RECEPTORS FOR CANCER IMMUNOTHERAPY**

(30) Priority: 07.03.2017 EP 17159754; 03.07.2017 EP 17179309
(62) Divisional of application: 18708695.4
(71) Applicant: Universität Basel, 4001 Basel (CH)
(72) Inventor: DE LIBERO, Gennaro, 4103 Bottmingen (CH); LEPORE, Marco, OX2 0EX Oxford Oxfordshire (GB); MORI, Lucia, 4103 Bottmingen (CH)
(74) Representative: Schulz Junghans Patentanwälte PartGmbB

(57) **Abstract**

The invention relates to a recombinant cell comprising an expression vector encoding specific T cell receptor protein heterodimers capable of recognizing an MR1 presented cancer antigen. It further provides recombinant cells expressing specific MR1 TCR for use in specific tumour entities.

The invention further relates to specific TCR proteins that bind to MR1, and to expression vectors encoding same.

## Description

The invention relates to the identification of tumour-reactive human T cell antigen receptors (TCRs) restricted to the non-polymorphic antigen-presenting molecule MR1. The functional TCR transcript sequences were isolated from clones representative of a novel population of human T cells (discovered by the inventors and termed MR1T cells) reacting to MR1-expressing tumour cells in the absence of any added foreign antigen and in MR1-dependent manner. The invention also relates to the use of MR1-restricted tumour-reactive TCR gene sequences in cancer treatment.

### Background of the invention

T lymphocytes can detect a diverse range of non-peptide antigens including lipids and phosphorylated isoprenoids, presented by non-polymorphic cell surface molecules. The heterogeneous phenotypic and functional properties of these T cells support specialized roles in host protection against infections, autoimmunity, and cancer. The repertoire of T cells specific for non-peptide antigens recently increased to include mucosal associated invariant T (MAIT) cells, which respond to small riboflavin precursors produced by a wide range of yeasts and bacteria, and presented by the MHC class I-related protein MR1. MAIT cells are frequent in human blood, kidney and intestine, and comprise a major fraction of T cells resident in the liver. Following activation, MAIT cells release an array of pro-inflammatory and immunomodulatory cytokines, and can mediate direct killing of microbe-infected cells. It remains unknown whether the role of MR1 extends beyond presentation of microbial metabolites to MAlT cells.

MR1 is a non-polymorphic MHC class I-like protein that is expressed at low levels on the surface of many cell types. MR1 is highly conserved across multiple species, with human and mouse MR1 sharing >90% sequence homology at the protein level.

The inventors proposed the existence of human T cells that recognize tumour-associated antigens presented by MR1. These novel T cells might participate in tumour immune surveillance, thus representing novel tools for cancer immunotherapy. Adoptive therapy with donor- or patient-derived T cells engineered to express TCRs specific for selected tumour-associated antigens represents a promising and safe strategy to induce clinically relevant anti-tumour immune response in cancer patients. Nevertheless, the majority of the so far identified tumour-associated antigens are peptides presented by polymorphic MHC molecules. The extreme polymorphism of MHC genes limits the application of this approach to those patients expressing unique MHC alleles. Targeting tumour-antigens bound to non-polymorphic antigen presenting molecules, such as MR1, might overcome this constraint and in principle be applicable to all patients bearing tumours expressing MR1. The use of tumour-reactive T cell receptors that recognize MR1-presented antigens might also have the advantage of complementing anti-tumour responses mediated by MHC-presented peptide antigens, excluding cross-competition of tumour antigens for binding to the same type of presenting molecule. In addition, this strategy may provide the possibility of targeting antigens of different nature on the same tumour cells, thus minimizing the potential occurrence of tumour escape variants under selective immune pressure. Therefore, the identification of MR1-presented tumour-associated antigens and the characterization of MR1-restricted TCRs recognizing these antigens might have important implications for cancer immunotherapy.

Based on this state of the art, the objective of the present invention is to provide novel means and methods of treatment for cancer. This objective is attained by the subject matter of the independent claims, with further advantageous solutions provided by the dependent claims, examples and figures disclosed herein.

### Definitions

The term *MR1* in the context of the present specification refers to either the MR1 gene (Entrez 3140) or the MR1 gene product (Uniprot Q95460).

The term *MR1T cell* in the context of the present specification refers to a T cell that expresses a T cell receptor capable of binding specifically to an MR1 molecule presented by a cancer cell.

The term *MR1T cell receptor* in the context of the present specification refers to a T cell receptor capable of binding specifically to an antigen presented by a cancer cell in association with an MR1 molecule.

In the present specification, the term *positive,* when used in the context of expression of a marker, refers to expression of an antigen assayed by a fluorescent labelled antibody, wherein the fluorescence is at least 30% higher (≥ 30 %), particularly ≥50% or ≥80%, in median fluorescence intensity in comparison to staining with an isotype-matched antibody which does not specifically bind the same target. Such expression of a marker is indicated by a superscript "plus" (⁺), following the name of the marker, e.g. CD4⁺.

In the present specification, the term *negative,* when used in the context of expression of a marker, refers to expression of an antigen assayed by a fluorescent labelled antibody, wherein the median fluorescence intensity is less than 30% higher, particularly less than 15% higher, than the median fluorescence intensity of an isotype-matched antibody which does not specifically bind the same target. Such expression of a marker is indicated by a superscript minus (⁻), following the name of the marker, e.g. CD127⁻.

The term *antibody* refers to whole antibodies including but not limited to immunoglobulin type G (IgG), type A (IgA), type D (IgD), type E (IgE) or type M (IgM), any antigen binding fragment or single chains thereof and related or derived constructs. The term encompasses a so-called nanobody or single domain antibody, an antibody fragment consisting of a single monomeric variable antibody domain.

### Summary of the invention

In one aspect, in the broadest sense, the invention relates to a method of treatment of cancer, wherein TCR sequences isolated from T cells reactive to MR1-expressing cancer cells (MR1T cells) are expressed after gene transfer in a population of patient T cells. These foreign, transgenically expressed TCR sequences are used for conferring specific recognition of MR1-expressing cancer cells to T cells as a treatment for the patient's tumour.

The invention similarly provides recombinant cells, particularly T cell preparations comprising a plurality of T cells, transduced with MR1T cell specific TCR genes. In certain embodiments, the T cells transduced with MR1T cell TCR genes can be used for adoptive cell immunotherapy in combination with other therapeutic interventions.

An aspect of the invention relates to a recombinant cell comprising an expression vector encoding specific T cell receptor protein heterodimers capable of recognizing an MR1 presented cancer antigen. It further provides recombinant cells expressing specific MR1 TCR for use in specific tumour entities.

A further aspect of the invention relates to specific TCR proteins that bind to MR1, and to expression vectors encoding same.

This specification also discloses a research method facilitating the identification of the TCR sequences isolated from T cells reactive to MR1-expressing cancer cells (MR1T cells) employed in the invention. This encompasses a method to isolate MR1-restricted T cells that recognize tumour-associated antigens. T cells from peripheral blood of normal donors or from cancer patients are stimulated with tumor cell lines representing the same type of tumor in patient. These tumor cell lines are transfected with the MR1 gene and thus express large amounts of MR1 protein on their plasma membrane. Activated T cells are sorted for expression of activation markers, (e.g. CD137, or CD150, or CD69, or ICOS) and are cloned as published (De Libero, Methods for the generation of T cell clones and epithelial cell lines from excised human biopsies or needle aspirates. In MHC 123-140 (IRL, Oxford; 1997)). Individual clones are tested for their capacity to recognize tumor cells in an MR1-restricted manner, for killing tumor cells and for release of inflammatory cytokines. The TCR genes of MR1-restricted and tumor-specific T cell clones are sequenced and identified.

This specification also discloses a method by which tumor-infiltrating T cells are prepared from the same cancer tissue biopsies according to our previously established protocol (De Libero, ibid.). Individual T cell clones are tested against a panel of tumor cell lines expressing MR1 protein. The most reactive T cell clones are studied for their MR1 restriction, tumor killing and release of inflammatory cytokines. The TCR genes of selected T cell clones are sequenced.

### Detailed description of the invention

A first aspect of the invention relates to a method of identifying and/or isolating a T cell that expresses a T cell receptor capable of binding specifically to a cancer antigen presented by a cancer cell in association with a non-polymorphic MHC I-related MR1 antigen-presenting molecule. This method comprises the steps of
a. providing a preparation of T cells isolated from a patient or a healthy donor, then
b. contacting, particularly co-culturing, this preparation of isolated T cells with cancer cells expressing MR1 protein in the absence of exogenous microbial-derived antigens in a contacting step, then
c. isolating a T cell that is specifically reactive to said cancer cells in an MR1-dependent manner in an isolation step.

MR1 in the physiological context of a non-tumour bearing patient presents bacterial riboflavin byproducts (above referred to as "exogenous microbial-derived antigens") and presents them to mucosal invariant T cells.

In certain embodiments, the contacting step comprises an expansion step, wherein the preparation of isolated T cells is expanded in the presence of cancer cells expressing MR1. In certain particular embodiments, the cancer cells are irradiated in order to prohibit their growth prior to being brought into contact with the T cells. This is going to be advantageous if the two cell types are meant to be kept in co-culture for extended periods of time and overgrowing of the culture by the cancer cells is to be avoided.

In other settings, particularly in clinical use (see below), where culturing times are short, the cancer cells may be used without irradiation.

In certain embodiments, the expansion step is conducted in the presence of IL-2, IL-7 and IL-15.

In certain embodiments, the isolation step comprises staining with one or more ligands, in particular one or more (monoclonal) antibodies specific for a cell surface marker selected from CD3, CD69, CD137, CD150, and / or ICOS). In particularly preferred embodiments the isolation step comprises selecting CD3⁺ CD137⁺, and/or CD3⁺ CD69⁺, and/or CD3⁺ CD150⁺, and/or CD3⁺ ICOS⁺ T cells, followed by flow cytometric analysis and cell sorting, particularly by using FACS or magnetic separation (MACS). Here positive expression (+) of a marker means at least 30% increase of the median fluorescence intensity over staining with isotype-matched antibody which does not specifically bind the same cell. In other words, T cells that express CD3 and CD137, and/or CD3 and CD69, and/or CD3 and CD150, and/or CD3 and ICOS are isolated using FACS or MACS. The skilled person is aware that in instances where the cells are isolated via FACS, cells that are positive for the expression of two (or more) different markers can be isolated in a single step. If magnetic separation is used, two separate steps have to be performed to isolate cells that are positive for the expression of two different markers.

The isolating step comprises selecting T cells that display MR1-restricted activity. In other words, this step comprises isolating T cells that are activated by an antigen presented on MR1.

In certain embodiments, the isolating step comprises selecting T cells that exhibit 2x increased expression of a cytokine selected from IFN-γ and/or GM-CSF release when stimulated with cells expressing MR1 compared to stimulation with cells not expressing MR1.

The skilled person is aware that an MR1-expressing cancer cell presents a particular cancer antigen, or a number of particular cancer antigens, on MR1.

In certain embodiments, the isolating step comprises selecting T cells that exhibit 2x increased expression of a cytokine selected from IFN-γ and/or GM-CSF release when stimulated with tumour cells expressing MR1 compared to stimulation with tumour cells (of the same origin, or same cell line) not expressing MR1.

Reactive cells are those that, in response to being contacted by an MR1-expressing cancer cell (presenting a cancer antigen in an MR1-restricted fashion), upregulate activation markers (particularly the markers cited in the preceding paragraphs), release cytokines and start to proliferate.

In other words, T cells that display MR1-restricted activity are T cells that can be activated by a tumour-associated antigen displayed by MR1.

These cells can be sorted by fluorescence activated cell sorting (FACS) after staining with the appropriate fluorescently labelled antibodies specific for the marker, or by sorting by magnetic beads labelled with the appropriate antibodies (which is the usual sorting method in a clinical setting).

In certain embodiments, the isolating step comprises expanding individual clones of the cells sorted as a function of their activation status, and then selecting T cell clones that display MR1-restricted activity, particularly cells that exhibit 2x increased expression of a cytokine selected from IFN-γ and/or GM-CSF when stimulated with cells expressing MR1 compared to stimulation with cells not expressing MR1.

In certain embodiments, the method further includes determining a nucleic acid sequence encoding a T cell receptor of the T cell isolated in the isolation step. In certain embodiments, the method includes determining two nucleic acid sequences encoding both T cell receptor chains of the T cell isolated in the isolation step.

Another aspect of the invention relates to a method of producing a preparation of transgenic MR1T cells reactive to MR1 in the absence of exogenous antigens. The method encompasses firstly, determining which T cell receptors are most likely to be reactive to a particular MR1-expressing cancer in a patient, then preparing a T cell population expressing these specific T cell receptor genes from expression constructs transferred into the cells, and administering these engineered T cells into the patient.

### This method comprising the steps of

a. providing a tumour sample obtained from a patient;
b. contacting said tumour sample with a plurality of MR1T cell receptor molecule reactive to MR1, either
   - presented on a plurality of T cell clones, wherein each T cell clone is characterized by an MR1T cell receptor molecule reactive to MR1; or
   - as soluble MR1T cell receptor molecules that are labelled, and their recognition is assayed in a non-cell-dependent fashion;
c. identifying a number of T cell clone(s) specifically reactive to said tumour sample;
d. providing a T cell preparation, particularly a T cell preparation obtained from the same patient;
e. introducing a nucleic acid expression construct encoding an MR1-reactive T cell receptor molecule expressed on a T cell clone identified as being specifically reactive to said tumour sample in step c into said T cell preparation, yielding a transgene T cell preparation

The transgene T cell preparation to the patient could thus be administered to the patient.

In certain embodiments, the said T cell preparation is obtained from the same patient (autologous adoptive T cell therapy). This method has the advantage of avoiding the risk of adverse reactions, particularly an allo-immune reaction driven by the endogenous T cell receptors of the engineered T cell preparation.

In certain embodiments, the said T cell preparation is obtained from another subject, particularly a HLA-matched subject (allogeneic adoptive T cell therapy). While depending on the quality of the HLA match, the risk of alloimmunity may be significant, the logistics and procedural advantages of having a large selection of pre-made TC preparations to select from may facilitate this therapy to a vastly larger patient community in comparison to the far higher costs and regulatory hurdles of a bespoke, patient-individual therapy.

Introduction of the MR1T cell receptor expression construct into the T cell preparation may be achieved by lentiviral transduction, which the inventors have routinely used in their work on MR1T cells, or by standard methods of DNA expression vector (plasmid) or RNA transfection. The skilled person is aware of the relevant protocols and procedures.

Optionally, the transgene T cell preparation may be kept in culture for some time prior to being administered to the patient in order to expand their number and, again optionally, to further stimulate their differentiation into a particularly desired T cell subset.

In certain embodiments, the T cell preparation obtained from said patient is obtained from peripheral blood of the patient, particularly wherein said T cell preparation is obtained by selecting peripheral blood mononuclear cells (PBMC) for expression of one or several T cell markers selected from the group containing CD4, CD8, CD27, CD45RA and CD57.

In certain embodiments, the T cell preparation obtained from said patient is obtained from a tumour biopsy followed by subsequent expansion in-vitro. In certain embodiments, T cells are expanded in the presence of phytohemagglutinin, IL-2, IL-7 and IL-15. Proliferating T cells are isolated by magnetic sorting and used for T cell receptor engineering or for cloning and isolation of tumour-specific MR1-restricted T cells. The isolated MR1T cells are used for TCR gene cloning.

The plurality of MR1-specific T cell clones can be prepared in advance of the procedure and held in form of a library or panel for ad-hoc use whenever the need for rapid characterization of a tumour arises. This step is essentially an identification of the MR1-specific T cell receptor molecules that will recognize a particular tumour entity.

Alternatively, soluble MR1T TCRs may be generated and multimerized (see Subbramanian et al. Nature Biotechnology, 22, 1429, (2004)). TCR multimers will be labelled with fluorochromes and used to stain tumour cells isolated from tumour biopsies. Binding of soluble MR1T TCR multimers will indicate the capacity of that MR1T TCR to recognize tumour cells and thus will facilitate selection of the MR1T TCRs suitable for gene therapy in that patient.

Another aspect of the invention relates to an expression vector comprising, and leading to the transcription of, a nucleic acid sequence encoding a functional T cell receptor heterodimer, or a T cell receptor α chain capable of forming a functional T cell receptor heterodimer together with a T cell receptor β chain, and/or a T cell receptor β chain capable of forming a functional T cell receptor heterodimer together with a T cell receptor α chain. Of note, also MR1-specific γ-δ heterodimers have been found by the inventors, so the same applies to these chains.

In embodiments where the expression vector comprises a nucleic acid sequence encoding a T cell receptor α chain or a T cell receptor β chain (or a γ or δ chain), two different expression vectors (one encoding an α chain (γ chain) and one encoding a β chain (δ chain)) have to be introduced into a cell in order to enable expression of a functional T cell receptor heterodimer by said cell. The T cell receptor heterodimer specifically binds to an MR1 molecule, wherein said MR1 molecule is expressed on a tumour cell and presents a tumour-associated antigen.

The expression of the above-mentioned nucleic acid sequences is controlled by a promoter sequence operable in a mammalian cell, particularly a human T-cell. In certain embodiments, the promoter is a constitutively activated promoter, for example the CMV immediate early promoter commonly used in molecular biology. In certain other embodiments, the promoter is an inducible promoter.

In certain embodiments of this aspect of the invention, the nucleic acid sequence comprised in the expression vector is or comprises a nucleic acid sequence that is selected from SEQ ID NOs 027 to 038, and/or encodes an amino acid sequence selected from SEQ ID NOs 001 to 012 (alpha chains).

In certain embodiments of this aspect of the invention, the nucleic acid sequence comprised in the expression vector is or comprises a nucleic acid sequence that is selected from SEQ ID NOs 039 to 050 and/or encodes an amino acid sequence selected from SEQ ID NOs 013 to 024 (beta chains).

In certain embodiments, the nucleic acid sequence encodes the T cell receptor γ chain encoded by SEQ ID NO 051 or encodes the T cell receptor γ chain specified by SEQ ID NO 025.

In certain embodiments, the nucleic acid sequence encodes the T cell receptor δ chain encoded by SEQ ID NO 052 or encodes the T cell receptor δ chain specified by SEQ ID NO 026.

Another aspect of the invention relates to a nucleic acid sequence encoding a functional T cell receptor heterodimer. The T cell receptor heterodimer specifically binds to a non-polymorphic MHC I-related (MR1) antigen-presenting molecule expressed on a tumour cell presenting a tumour-associated antigen.

In certain embodiments, the nucleic acid sequence encodes a T cell receptor α chain and is selected from SEQ ID NOs 027 to 038, or encodes a T cell receptor α chain specified by an amino acid sequence selected from SEQ ID NOs 001 to 012.

In certain embodiments, the nucleic acid sequence encodes a T cell receptor β chain and is selected from SEQ ID NOs 039 to 050 or encodes a T cell receptor β chain specified by an amino acid sequence selected from SEQ ID NOs 013 to 024.

In certain embodiments, the MR1 T cell receptor is constituted of one alpha chain and one beta chain disclosed herein. The inventors have surprisingly found that the alpha and beta chains may be combined to render functional TCR molecules capable of recognizing MR1.

In certain embodiments, the MR1 T cell receptor is constituted of one alpha chain and one beta chain as specified by the sequences of the following list:
a. SEQ ID NOs 001 and 023,
b. SEQ ID NOs 002 and 022,
c. SEQ ID NOs 003 and 021,
d. SEQ ID NOs 004 and 020,
e. SEQ ID NOs 005 and 019,
f. SEQ ID NOs 006 and 017,
g. SEQ ID NOs 007 and 018,
h. SEQ ID NOs 008 and 016,
i. SEQ ID NOs 009 and 015,
j. SEQ ID NOs 010 and 014,
k. SEQ ID NOs 011 and 013,
l. SEQ ID NOs 012 and 024, or
m. SEQ ID NOs 025 and 026,

Another aspect of the invention relates to a T cell receptor protein that binds to a non-polymorphic MHC I-related MR1 antigen-presenting molecule. The MR1 molecule is expressed on a tumour cell and presents a tumour-associated antigen. In certain embodiments, the T cell receptor protein that binds to a non-polymorphic MHC I-related MR1 antigen-presenting molecule is identified by the method according to the first aspect of the invention.

In certain embodiments, the T cell receptor protein comprises a T cell receptor α chain characterized by an amino acid sequence selected from SEQ ID NOs 001 to 012 and a T cell receptor β chain characterized by an amino acid sequence selected from SEQ ID NOs 013 to 024.

In certain embodiments, the T cell receptor protein comprises a T cell receptor γ chain characterized by the amino acid sequence SEQ ID NO 25 and a T cell receptor δ chain characterized by the amino acid sequence SEQ ID NOs 26.

Another aspect of the invention relates to a recombinant cell comprising the expression vector according to the invention, and/or the T cell receptor polypeptide according to the invention as specified in the preceding paragraphs. The skilled person is aware that in instances where the expression vector only comprises a nucleic acid sequence encoding a T cell receptor α chain (or a γ chain) or a T cell receptor β chain (or a δ chain), but not both, two different expression vectors (one encoding an α/γ chain and one encoding a β/δ chain) have to be introduced into the recombinant cell in order to enable expression of a functional T cell receptor heterodimer by said cell. In certain embodiments, the recombinant cell is a T cell derived from peripheral blood. In certain embodiments, the recombinant cell is derived from a tumour infiltrating lymphocyte.

Yet another aspect of the invention relates to the use of the recombinant cell according to the previously specified aspect of the invention for use in a method of therapy or prevention of cancer. The method comprises administration of the recombinant cell.

In certain embodiments, the cancer is characterized by MR1 expression.

In certain embodiments, the administration is effected by adoptive T cell immunotherapy.

The invention further relates to a method of treatment, or prevention of recurrence, of cancer, comprising administration of the recombinant cell according to the invention. In certain embodiments, the cancer is characterized by MR1 expression.

In certain embodiments, the administration is achieved by adoptive T cell immunotherapy.

The invention also relates to a collection of nucleic acid sequences, wherein each member of the collection encodes a different T cell receptor α chain, T cell receptor β chain, T cell receptor γ chain, T cell receptor δ chain or a T cell receptor α chain and β chain combination, or a T cell receptor γ chain and δ chain combination, wherein said combination is capable of specifically binding to an MR1 molecule presenting a cancer antigen. The nucleic acid sequences are capable to facilitate the expression of the T cell receptor α chain, β chain, or α and β chain combination in a mammalian cell.

Such collection will be used to select transgene constructs for transfer into T cells collected from a patient. After identification of the TCR sequences that are best to fit instigate reaction to a particular set of tumour antigens presented by the tumour in the first phase of the method of treatment, the physician will need to be able to select pre-produced expression vectors from such collection manufactured under GMP, to quickly effect the gene transfer into the patient's T cells.

In certain embodiments, the collection comprises a sequence selected from SEQ ID NO 27 to SEQ ID NO 52 and/or the collection comprises sequences encoding a T cell receptor molecule (or a T cell receptor constituting α or β, or γ or δ, chain) selected from SEQ ID NO 1 to SEQ ID NO 26.

Yet another aspect of the invention relates to a collection of recombinant T cells, wherein each member of the collection expresses as a transgene a T cell receptor capable of specifically binding to an MR1 molecule presenting a cancer antigen. In certain embodiments, the collection comprises a recombinant T cell comprising a T cell receptor protein heterodimer according to the respective aspect of the invention.

The inventors identified and isolated a novel population of human MR1-restricted T cells reactive to a variety of tumour cells in MR1-dependent manner. MR1T cell clones were commonly found in the blood of different healthy individuals, expressed diverse TCR genes and did not recognize previously identified microbial or folate-derived ligands of MR1. Instead, they recognized diverse sets of yet unknown antigens isolated from tumour cells and presented by MR1. The identification and characterization of the stimulatory antigens associated with tumour cells is currently ongoing. MR1T cell clones recognized and killed different types of tumour cells, thus displaying marked anti-tumour activity *in vitro.* In addition, they released different combinations of Th1, Th2 and Th17 cytokines, and displayed multiple chemokine receptor expression profiles, suggesting phenotypical and functional heterogeneity. Importantly, when paired TCR α and β genes or TCR γ and δ genes isolated from individual MR1T cell clones were transferred into TCR-deficient T cells, the recipient T cells acquired the capacity to recognize MR1-expressing tumour cells, thus indicating that the MR1T cell TCR gene transfer is sufficient for this type of tumour recognition and might be used to instruct select T cells to recognize MR1-expressing tumour cells.

Taken together, these findings reveal a novel functionally diverse population of tumour-reactive human T cells restricted to non-polymorphic MR1 molecules with diverse potential role in tumour immunity, thus providing new conceptual frameworks for cancer immune surveillance and immunotherapies.

In the present specification, the following abbreviations are used: APC, antigen-presenting cell; β2m, β2 microglobulin; DC, dendritic cell; GM-CSF, granulocyte-macrophage colony-stimulating factor; HPLC, high-pressure liquid chromatography; IFN-γ, interferon-γ; mAb, monoclonal antibody; MAIT cell, mucosal associated invariant T cell; MHC, major histocompatibility complex; MR1, MHC class I-related molecule; MR1T cell, MR1-restricted T cell; PBMC, peripheral blood mononuclear cell; TCR, T cell receptor; TIL, tumour-infiltrating lymphocyte.

The invention further encompasses the following items:
1. A method of isolating a T cell that expresses a T cell receptor capable of binding specifically to an antigen presented by a cancer cell in association with an MR1 molecule, said method comprising the steps of
   a. providing a preparation of T cells, then
   b. contacting said preparation of T cells with a cancer cell expressing MR1 in a contacting step, then
   c. isolating a T cell that is specifically reactive to said cancer cell in an isolation step.
2. The method according to item 1, wherein said contacting step comprises an expansion step, wherein said preparation of isolated T cells is expanded in the presence of a cancer cell expressing MR1.
3. The method according to item 1 or 2, wherein said expansion step is conducted in the presence of IL-2, and/or IL-7 and/or IL-15.
4. The method according to any one of the previous items, wherein said isolation step comprises staining the T cell preparation with a ligand specific for a cell surface marker selected from CD3, CD69, CD137, CD150, and/or ICOS, particularly wherein said isolation step comprises selecting CD3⁺ CD137⁺, and/or CD3⁺ CD69⁺, and/or CD3⁺ CD150⁺, and/or CD3⁺ ICOS⁺ T cells, followed by flow cytometric analysis and cell sorting, particularly by using FACS or magnetic separation.
5. The method according to any one of the previous items, wherein said ligand specific for a cell surface marker is an antibody or antibody-like molecule.
6. The method according to any one of the previous items, wherein said isolating step comprises selecting T cells that exhibit 2x increased expression of a cytokine selected from IFN-γ and/or GM-CSF release when stimulated with cells expressing MR1, compared to stimulation with cells not expressing MR1.
7. The method according to any one of the previous items, further including determining a nucleic acid sequence encoding a T cell receptor of the T cell isolated in the isolation step.
8. A method of preparing a preparation of MR1T cells that express a T cell receptor capable of binding to an antigen presented by a cancer cell in association with an MR1 molecule, comprising the steps of
   a. providing a tumour sample obtained from a patient;
   b. contacting said tumour sample with
      i. a plurality of T cell clones, wherein each T cell clone is characterized by an MR1T cell receptor molecule capable of binding specifically to an antigen presented by a cancer cell in association with an MR1 molecule; or
      ii. a plurality of labelled and multimerized soluble TCRs isolated from MR1T cell receptor molecules.
   c. identifying an MR1T cell receptor specifically reactive to said tumour sample;
   d. providing a T cell preparation;
   e. introducing a nucleic acid expression construct encoding an MR1-reactive T cell receptor molecule expressed on a T cell clone identified as being specifically reactive to said tumour sample in step c into said T cell preparation, yielding a transgene T cell preparation.
9. The method according to item 8, wherein said T cell preparation is obtained from the same patient (autologous adoptive T cell therapy).
10. The method according to item 8, wherein said T cell preparation is obtained from another subject, particularly a HLA-matched subject (allogeneic adoptive T cell therapy).
11. The method according to item 8, wherein said T cell preparation obtained from said patient is obtained from peripheral blood of the patient, particularly wherein said T cell preparation is obtained by selecting PBMC for expression of one or several T cell markers selected from the group containing CD4, CD8, CD27, CD45RA and CD57, particularly selecting CD3⁺ CD4⁺, or CD3⁺ CD8⁺, or CD3⁺ CD27⁺ CD45RA⁺, or CD3⁺ CD27⁺ CD45RA⁻, or CD3⁺ CD27⁻ CD45RA⁻, or CD3⁺ CD57⁻ or CD3⁺ CD57⁺ T cells.
12. The method according to item 8, wherein said T cell preparation obtained from said patient is obtained from a tumour biopsy followed by subsequent expansion in-vitro.
13. A preparation of MR1-specific T cells obtained by the method of any one of item 8 to 12 for use in a method of therapy or prevention of cancer, in particular a cancer characterized by MR1 expression.
14. An expression vector comprising a nucleic acid sequence encoding
   a. a functional T cell receptor heterodimer, or
   b. a T cell receptor α chain capable of forming a functional T cell receptor heterodimer together with a T cell receptor β chain, and/or
   c. a T cell receptor β chain capable of forming a functional T cell receptor heterodimer together with a T cell receptor α chain, or
   d. a T cell receptor γ chain capable of forming a functional T cell receptor heterodimer together with a T cell receptor δ chain, and/or
   e. a T cell receptor δ chain capable of forming a functional T cell receptor heterodimer together with a T cell receptor γ chain,
   wherein said T cell receptor heterodimer specifically binds to an MR1 molecule, wherein said MR1 molecule is expressed on a tumour cell and presents a tumour-associated antigen, and wherein particularly said nucleic acid sequence
   i. is or comprises a nucleic acid sequence selected from SEQ ID NOs 027 to 038, and/or encodes an amino acid sequence selected from SEQ ID NOs 001 to 012 and/or
   ii. is or comprises a nucleic acid sequence selected from SEQ ID NOs 039 to 050 and/or encodes an amino acid sequence selected from SEQ ID NOs 013 to 024, or
   iii. is or comprises the nucleic acid sequence of SEQ ID NO 051 and/or encodes the amino acid sequence of SEQ ID NO 025, and/or
   iv. is or comprises the nucleic acid sequence of SEQ ID NO 052 and/or encodes the amino acid sequence of SEQ ID NO 026.
15. An isolated T cell receptor protein heterodimer that binds to an MR1 molecule, wherein said MR1 molecule is expressed on a tumour cell, particularly wherein the T cell receptor protein heterodimer has been identified by a method according to any one of items 1 to 7.
16. The isolated T cell receptor protein heterodimer that binds to an MR1 molecule according to item 15, wherein said MR1 molecule presents a tumour-associated antigen.
17. The isolated T cell receptor protein heterodimer according to item 15 or 16, wherein said isolated T cell receptor protein comprises an amino acid sequence selected from SEQ ID NOs 001 to 012 and an amino acid sequence selected from SEQ ID NOs 013 to 024, or the amino sequences of SEQ ID NO 025 and 026.
18. The isolated T cell receptor protein heterodimer according to item 17, wherein said isolated T cell receptor protein comprises a pair of amino acid sequence selected from
   a. SEQ ID NOs 001 and 023,
   b. SEQ ID NOs 002 and 022,
   c. SEQ ID NOs 003 and 021,
   d. SEQ ID NOs 004 and 020,
   e. SEQ ID NOs 005 and 019,
   f. SEQ ID NOs 006 and 017,
   g. SEQ ID NOs 007 and 018,
   h. SEQ ID NOs 008 and 016,
   i. SEQ ID NOs 009 and 015,
   j. SEQ ID NOs 010 and 014,
   k. SEQ ID NOs 011 and 013,
   l. SEQ ID NOs 012 and 024, or
   m. SEQ ID NOs 025 and 026.
19. A recombinant cell comprising the expression vector according to item 14 or the T cell receptor protein heterodimer according to any one of items 15 to 18, wherein said recombinant cell is a T cell derived from
   a. peripheral blood or
   b. a tumour infiltrating lymphocyte.
20. The recombinant cell according to item 18 for use in a method of therapy or prevention of cancer, in particular a cancer characterized by MR1 expression.
21. The recombinant cell for use in a method of therapy or prevention of cancer according to item 20, wherein said cell is administered by adoptive T cell immunotherapy.
22. A collection of nucleic acid sequences, wherein each member of the collection facilitates the expression of a different T cell receptor α chain, T cell receptor β chain, or a T cell receptor α chain and β chain combination in a mammalian cell, wherein said combination is capable of specifically binding to an MR1 molecule presenting a cancer antigen.
23. The collection of nucleic acid sequences according to item 22, wherein the collection comprises a sequence selected from SEQ ID NO 027 to SEQ ID NO 052 and/or the collection comprises sequences encoding a T cell receptor molecule (or a T cell receptor constituting alpha, beta, gamma or delta chain) selected from SEQ ID NO 001 to SEQ ID NO 026.
24. A collection of recombinant T cells, wherein each member of the collection expresses as a transgene a T cell receptor capable of specifically binding to an MR1 molecule presenting a cancer antigen,
25. The collection of T cells according to item 24, wherein the collection comprises a cell comprising a T cell receptor molecule according to item 15 to 18.
26. An MR1 expressing nucleic acid expression vector comprising a nucleic acid sequence encoding MR1 under control of a promoter sequence operable in a mammalian cell, for use in cancer treatment.
27. The MR1 expressing nucleic acid expression vector for use in cancer treatment according to item 26, wherein said MR1 expressing nucleic acid expression vector is administered before, concomitant with or after administration of
   a. a recombinant cell comprising the expression vector according to item 14 or the T cell receptor protein heterodimer according to any one of items 15 to 18, and/or
   b. a preparation of MR1-specific T cells obtained by the method of any one of items 8 to 12 and/or
   c. an expression vector according to item 14.

A. A method of isolating a T cell that expresses a T cell receptor capable of binding specifically to an antigen presented by a cancer cell in association with an MR1 molecule (Uniprot Q95460), said method comprising the steps of
   a. providing a preparation of T cells, then
   b. contacting said preparation of T cells with a cancer cell expressing MR1 in a contacting step, then
   c. isolating a T cell that is specifically reactive to said cancer cell in an isolation step.
B. The method according to item A wherein said contacting step comprises an expansion step, wherein said preparation of isolated T cells is expanded in the presence of a cancer cell expressing MR1.
C. The method according to any one of the previous items A or B, further including determining a nucleic acid sequence encoding a T cell receptor of the T cell isolated in the isolation step.
D. A method of preparing a preparation of MR1T cells that express a T cell receptor capable of binding to an antigen presented by a cancer cell in association with an MR1 molecule, comprising the steps of
   a. providing a tumour sample obtained from a patient;
   b. contacting said tumour sample with
      i. a plurality of T cell clones, wherein each T cell clone is characterized by an MR1T cell receptor molecule capable of binding specifically to an antigen presented by a cancer cell in association with an MR1 molecule; or
      ii. a plurality of labelled and multimerized soluble TCRs isolated from MR1T cell receptor molecules.
   c. identifying an MR1T cell receptor specifically reactive to said tumour sample;
   d. providing a T cell preparation;
   e. introducing a nucleic acid expression construct encoding an MR1-reactive T cell receptor molecule expressed on a T cell clone identified as being specifically reactive to said tumour sample in step c into said T cell preparation, yielding a transgene T cell preparation.
E. A preparation of MR1-specific T cells obtained by the method of item D for use in a method of therapy or prevention of cancer, in particular a cancer characterized by MR1 expression.
F. A vector comprising a nucleic acid sequence encoding
   a. a functional T cell receptor heterodimer, or
   b. a T cell receptor α chain capable of forming a functional T cell receptor heterodimer together with a T cell receptor β chain, and/or
   c. a T cell receptor β chain capable of forming a functional T cell receptor heterodimer together with a T cell receptor α chain, or
   d. a T cell receptor γ chain capable of forming a functional T cell receptor heterodimer together with a T cell receptor δ chain, and/or
   e. a T cell receptor δ chain capable of forming a functional T cell receptor heterodimer together with a T cell receptor γ chain,
   wherein said T cell receptor heterodimer specifically binds to an MR1 molecule, wherein said MR1 molecule is expressed on a tumour cell and presents a tumour-associated antigen,
   and wherein particularly said nucleic acid sequence
   i. is or comprises a nucleic acid sequence selected from SEQ ID NOs 027 to 038, and/or encodes an amino acid sequence selected from SEQ ID NOs 001 to 012 and/or
   ii. is or comprises a nucleic acid sequence selected from SEQ ID NOs 039 to 050 and/or encodes an amino acid sequence selected from SEQ ID NOs 013 to 024 or
   iii. is or comprises the nucleic acid sequence of SEQ ID NO 051 and/or encodes the amino acid sequence of SEQ ID NO 025, and/or iv. is or comprises the nucleic acid sequence of SEQ ID NO 052 and/or encodes the amino acid sequence of SEQ ID NO 026.
G. An isolated T cell receptor protein heterodimer that binds to an MR1 molecule, wherein said MR1 molecule is expressed on a tumour cell, particularly wherein the T cell receptor protein heterodimer has been identified by a method according to any one of items A to C.
H. The isolated T cell receptor protein heterodimer that binds to an MR1 molecule according to item G, wherein said MR1 molecule presents a tumour-associated antigen.
I. The isolated T cell receptor protein heterodimer according to item G or H, wherein said isolated T cell receptor protein comprises an amino acid sequence selected from SEQ ID NOs 001 to 012 and an amino acid sequence selected from SEQ ID NOs 013 to 024, or the amino sequences of SEQ ID NO 025 and 026, particularly wherein said isolated T cell receptor protein comprises a pair of amino acid sequence selected from
   a. SEQ ID NOs 001 and 023,
   b. SEQ ID NOs 002 and 022,
   c. SEQ ID NOs 003 and 021,
   d. SEQ ID NOs 004 and 020,
   e. SEQ ID NOs 005 and 019,
   f. SEQ ID NOs 006 and 017,
   g. SEQ ID NOs 007 and 018,
   h. SEQ ID NOs 008 and 016,
   i. SEQ ID NOs 009 and 015,
   j. SEQ ID NOs 010 and 014,
   k. SEQ ID NOs 011 and 013,
   l. SEQ ID NOs 012 and 024, or
   m. SEQ ID NOs 025 and 026.
J. A recombinant cell comprising the vector according to item F or the T cell receptor protein heterodimer according to any one of item G to I, wherein said recombinant cell is a T cell derived from
   a. peripheral blood or
   b. a tumour infiltrating lymphocyte.
K. The recombinant cell according to item J for use in a method of therapy or prevention of cancer, in particular a cancer characterized by MR1 expression.
L. A collection of nucleic acid sequences, wherein each member of the collection facilitates the expression of a different T cell receptor α chain, T cell receptor β chain, or a T cell receptor α chain and β chain combination in a mammalian cell, wherein said combination is capable of specifically binding to an MR1 molecule presenting a cancer antigen, wherein the collection comprises a sequence selected from SEQ ID NO 027 to SEQ ID NO 052 and/or the collection comprises sequences encoding a T cell receptor molecule (or a T cell receptor constituting alpha, beta, gamma or delta chain) selected from SEQ ID NO 001 to SEQ ID NO 026.
M. A collection of recombinant T cells, wherein each member of the collection expresses as a transgene a T cell receptor capable of specifically binding to an MR1 molecule presenting a cancer antigen, wherein the collection comprises a cell comprising a T cell receptor molecule according to item G to I.

The invention is further illustrated by the following examples and figures, from which further embodiments and advantages can be drawn. These examples are meant to illustrate the invention but not to limit its scope.

### Brief description of the Figures

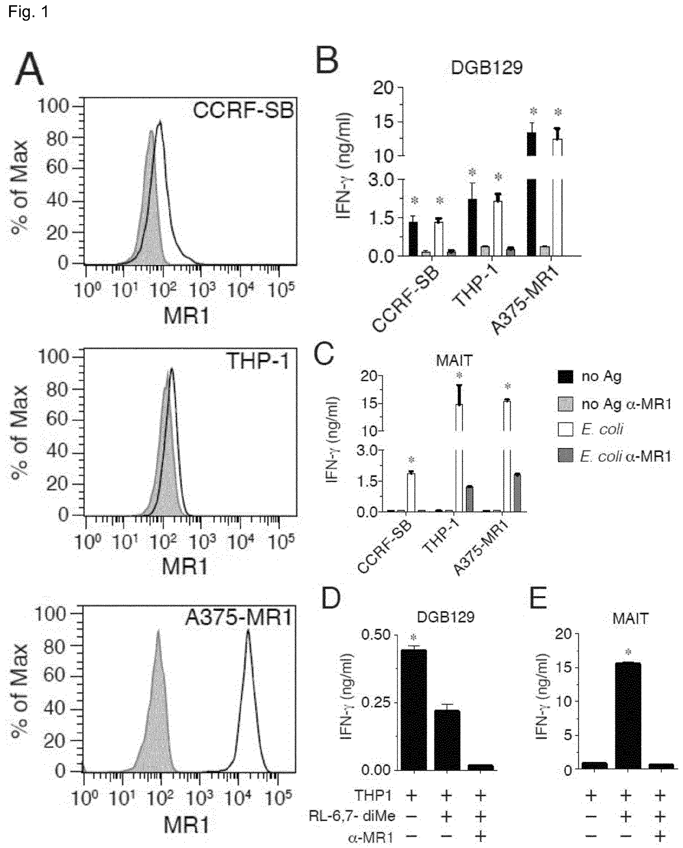

**Figure 1****.** MR1T cells do not recognize microbial antigens. (**A**) Surface expression of MR1 by CCRFSB, THP-1 and A375-MR1 cells. Grey histograms indicate staining with isotype-matched control mAbs. Stimulation of (**B**) MR1T cell clone DGB129 or (**C**) MAIT cell clone SMC3 by the three cell lines in A in the absence (no Ag) or presence of *E. coli* lysate (*E. coli*) and/or anti-MR1 blocking mAbs (α-MR1). The MAlT clone SMC3 was previously isolated from PBMC of a healthy donor and expresses canonical MAIT phenotype and function. Columns indicate IFN-γ release (mean + SD). Stimulation of (**D**) DGB129 MR1T or (**E**) SMC3 MAIT cells by THP-1 cells, constitutively expressing surface MR1, loaded with synthetic 6,7-dimethyl-8-D-ribityllumazine (RL-6,7-diMe) with or without anti-MR1 mAbs. Columns indicate mean IFN-γ release + SD. Data are representative of four (A, B and C), two (D and E). * P<0.05 (Unpaired Student's t-test).

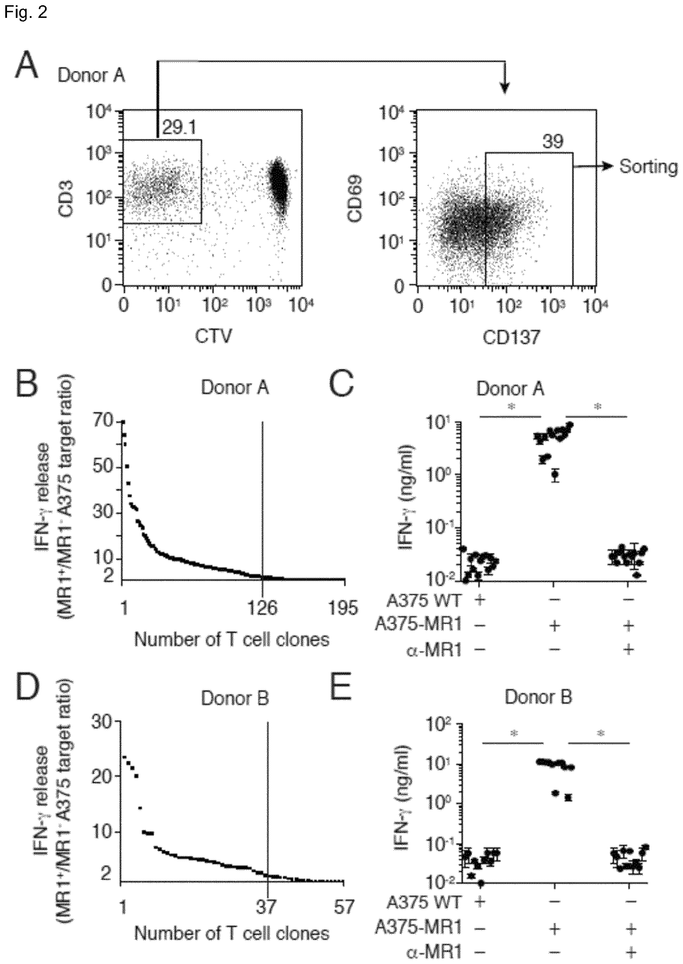

**Figure 2****.** Isolation strategy of MR1T cell clones from peripheral T cells. (**A**) FACS analysis of purified T cells previously expanded with irradiated A375-MR1 cells following overnight co-culture with A375-MR1 cells in the absence of exogenous antigens. Left dot plot shows CD3 and CellTrace violet (CTV) staining in live cells. Right dot plot shows CD69 and CD137 expression of CD3-positive CTV-negative gated cells. Arrows indicate gating hierarchy. Numbers indicate the percentages of cells within the gates. Cells from Donor A are illustrated as a representative donor. (**B**, **D**) Cumulative results of T cell clones screening from Donors A and B. T cell clones were generated from CD3⁺CTV⁻CD137⁺ sorted T cells as depicted in **A.** Graphs show the individual clones (x axis) and their IFN-γ release (y axis), expressed as ratio between the amount of cytokine secreted in response to A375-MR1 cells vs. A375 WT cells. Each dot represents a single T cell clone, tested at the same time in the indicated experimental conditions. The vertical lines indicate the number of T cell clones displaying MR1-restricted reactivity (*i*.*e*. the clones showing an IFN-γ release ratio above the arbitrary cut-off of 2). Results are representative of two independent experiments. (**C**, **E**) IFN-γ release by 14 representative clones from Donor A and 11 clones from Donor B after stimulation with A375 WT, A375-MR1 and A375-MR1 in the presence of blocking anti-MR1 mAbs (α-MR1). Dots represent the IFN-γ release (mean ± SD of duplicate cultures) by each clone. Results are representative of three independent experiments. * P<0.05 (Unpaired Student's t-test).

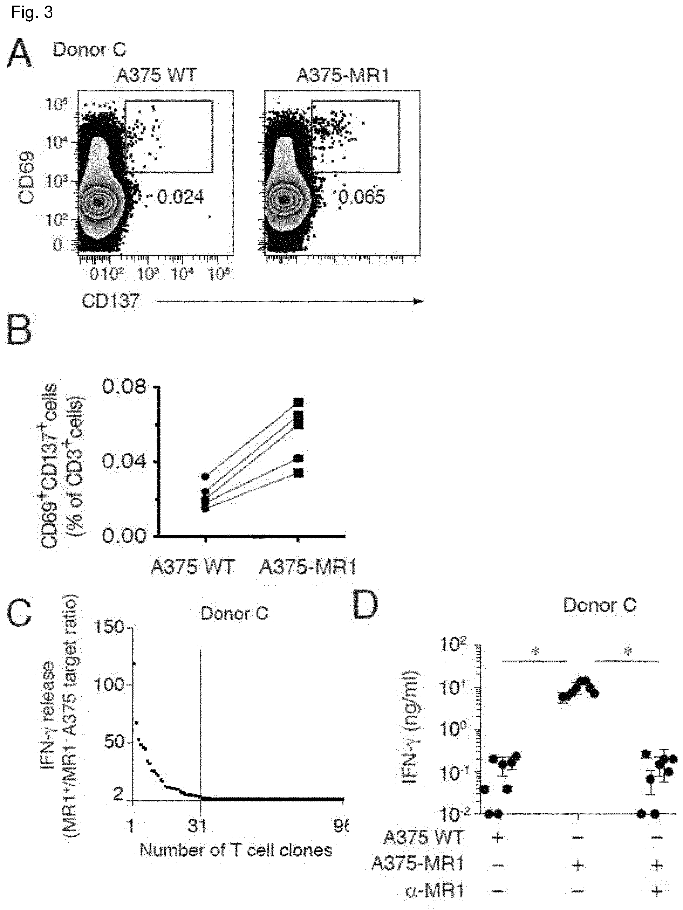

**Figure 3****.** MR1T cells are common in the blood of healthy individuals. (**A**) Flow cytometry analysis of purified T cells from a representative donor (Donor C) after overnight co-culture with A375 WT or A375-MR1 cells. Dot plots show CD69 and CD137 expression on live CD3⁺ cells. Numbers indicate the percentage of cells in the gates. (**B**) Frequency of CD69⁺CD137⁺ T cells from 5 different donors after overnight co-culture with A375 WT or A375-MR1 cells. (**C**) Cumulative results of T cell clone stimulation assays from Donor C. T cell clones were generated from CD3⁺CD69⁺CD137⁺ sorted T cells as depicted in A, right dot plot. The graph shows the number of tested clones (x axis) and IFN-γ release (y axis) expressed as ratio between the amounts of cytokine secreted in response to A375-MR1 cells vs. A375 WT cells. Each dot represents a single T cell clone, tested at the same time in the indicated experimental conditions. The vertical line indicates the number of T cell clones displaying MR1-restricted reactivity (*i*.*e*. the clones showing an IFN-γ release ratio above arbitrary cut-off of 2). Results are representative of two independent experiments. (**D**) Recognition of A375-MR1 but not A375 WT cells in the absence of exogenous antigens by 8 representative MR1-restricted T cell clones from Donor C. Inhibition of T cell clone reactivity to A375-MR1 cells by blocking anti-MR1 mAbs (α-MR1). Dots represent the IFN-γ release (mean ± SD of duplicate cultures) by each clone tested in the three experimental conditions. Results are representative of three independent experiments. * P<0.05 (Unpaired Student's t-test).

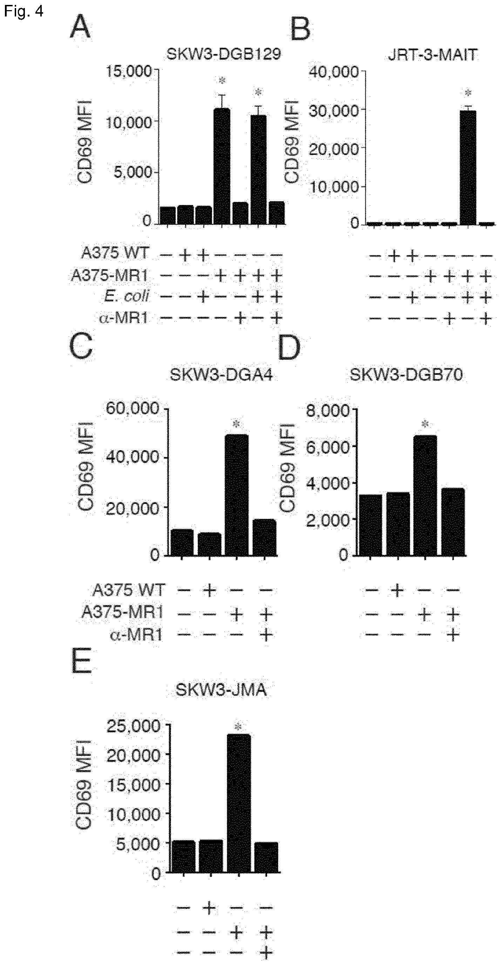

**Figure 4****. MR1T TCR gene transfer confers MR1-restricted recognition of A375 cells.** Stimulation of (**A**) SKW-3 cells expressing the DGB129 TCR (SKW3-DGB129) and (**B**) J.RT3-T3.5 cells expressing the MAlT MRC25 TCR (J.RT3-MAIT) with A375 cells that expressed (A375-MR1) or lacked (A375 WT) MR1, with or without *E. coli* lysate and anti-MR1 mAbs. Stimulation of SKW-3 cells expressing the TCRs of three individual MR1T cell clones (**C**) DGA4 (SKW3-DGA4), (D) DGB70 (SKW3-DGB70) and (**E**) JMA (SKW3-JMA) with A375-MR1 or A375 WT cells in the presence or not of or anti-MR1 mAbs. CD69 median fluorescence intensity (MFI) + SD of duplicate cultures of transduced T cells are shown. The CD69 MFI of transduced T cells cultured in the absence of APCs is also shown. Mock-transduced T cells showed background levels of CD69 expression when incubated with A375-MR1 or A375 WT (not shown). Data are representative of three independent experiments. * P<0.05 (Unpaired Student's t-test).

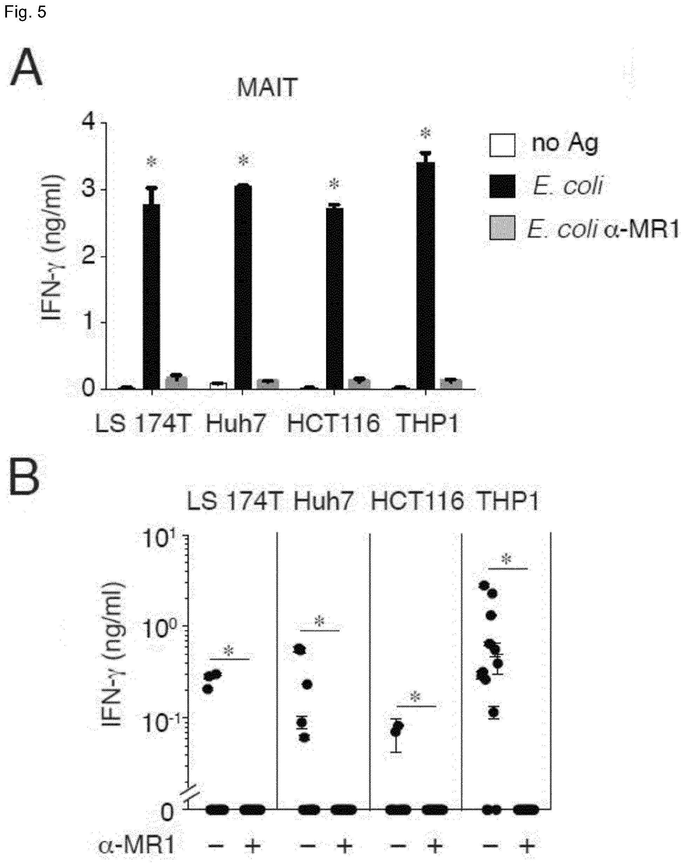

**Figure 5****.** Differential recognition of various types of tumour cells by MR1T cell clones. (**A**) Recognition of four human cells lines expressing constitutive surface levels of MR1 by the representative SMC3 MAIT cell clone in the absence (no Ag) or presence of *E. coli* lysate (*E. coli*) with or without anti-MR1 blocking mAbs (α-MR1). (**B**) Recognition of the same cell types as in **A** by thirteen MR1T cell clones with or without anti-MR1 mAbs (α-MR1). Graphs show IFN-γ release (mean ± SD of duplicate cultures).

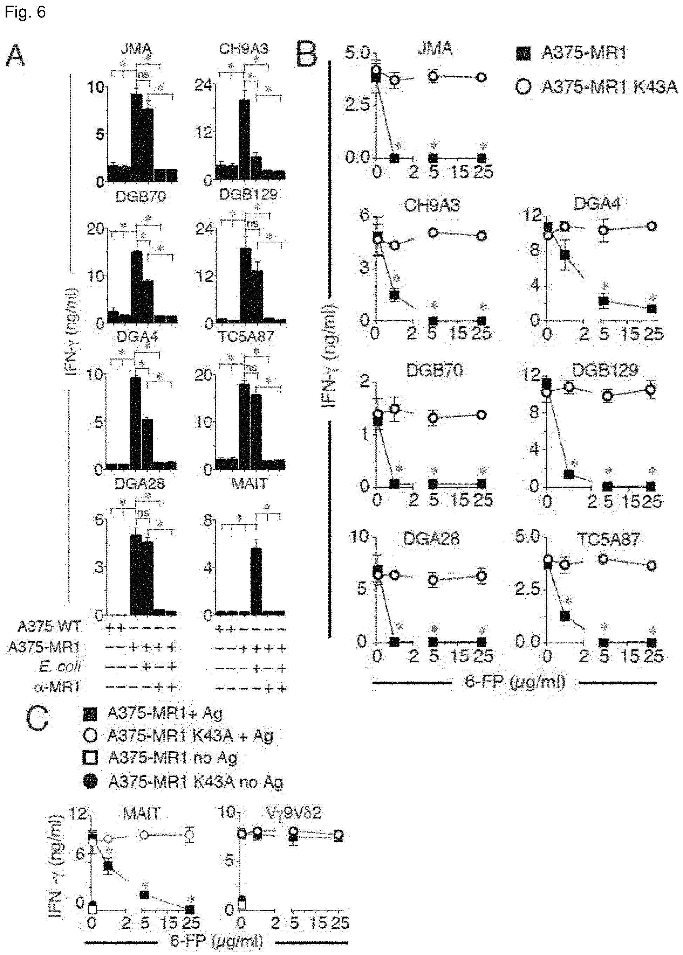

**Figure 6****.** MR1T cell clones do not react to microbial ligands or to 6-FP. (**A**) Response of seven MR1T cell clones and one control MAIT cell clone co-cultured with A375 cells expressing (A375-MR1) or not (A375 WT) MR1 in the presence or absence of *E. coli* lysate. Blocking of T cell clone reactivity by anti-MR1 mAbs (α-MR1) is also shown. (**B**) Response of MR1T cell clones to A375 cells expressing either WT MR1 molecules (A375-MR1) or K43A-mutated MR1 molecules (A375-MR1 K43A) in the presence of 6-formyl pterin (6-FP). (**C**) Stimulation of control MAIT cell clone MRC25 or control TCR Vγ9Vδ2 clone G2B9 with A375-MR1 or A375-MR1 K43A cells previously incubated with or without *E. coli* lysate or zoledronate, respectively, either in the absence or presence of 6-FP. Results are expressed as mean ± SD of IFN-γ measured in duplicate cultures. Results are representative of three independent experiments. * P<0.05 (Unpaired Student's t-test).

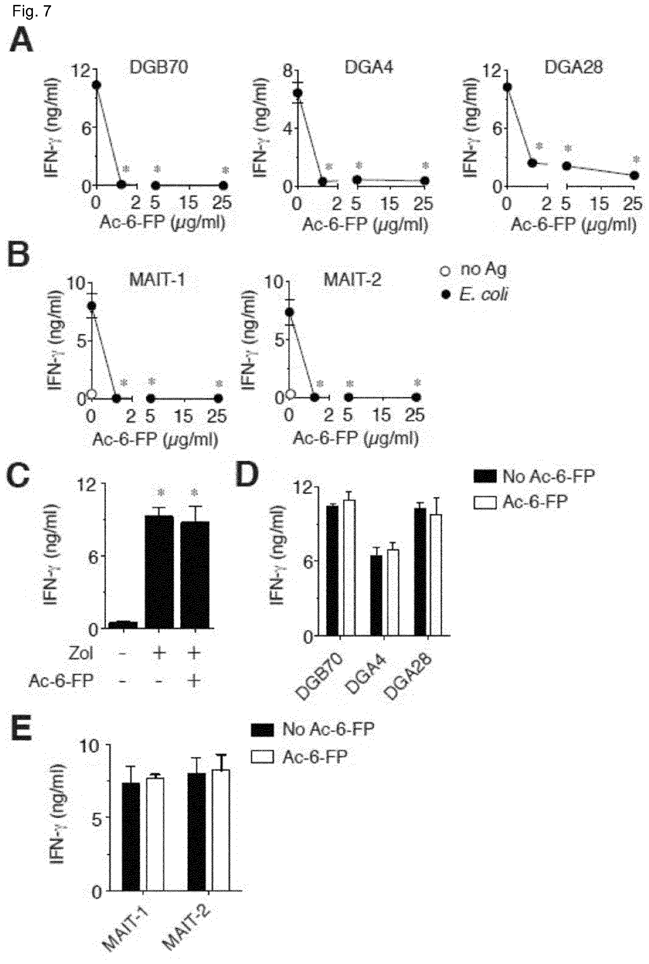

**Figure 7****.** MR1T cell clones do not recognize Ac-6-FP. (**A**) Stimulation of three representative MR1T cell clones by A375-MR1 cells in the absence or presence of acetyl-6-formyl pterin (Ac-6-FP). (**B**) Stimulation of two MAlT cell clones (MRC25 and SMC3) by A375-MR1 cells pulsed with *E. coli* lysate in the absence or presence of Ac-6-FP. (**C**) A375-MR1 cells were treated with zoledronate (Zol) in the absence or presence of Ac-6-FP (25 µg/ml) and used to stimulate a TCR Vγ9-Vδ2 cell clone (G2B9). (**D**) A375 cells expressing K43A mutant MR1 molecules (A375-MR1 K43A) were used to stimulate the three MR1T cell clones shown in **A,** in the absence or presence of Ac-6-FP (25 µg/ml). (**E**) Stimulation of the two MAlT cell clones used in **B** by A375-MR1 K43A cells pulsed with *E. coli* lysate in the absence or presence of Ac-6-FP (25 µg/ml). Results are expressed as mean ± SD of IFN-γ release assessed in duplicate cultures and are representative of three independent experiments. * P<0.05 (Unpaired Student's t-test).

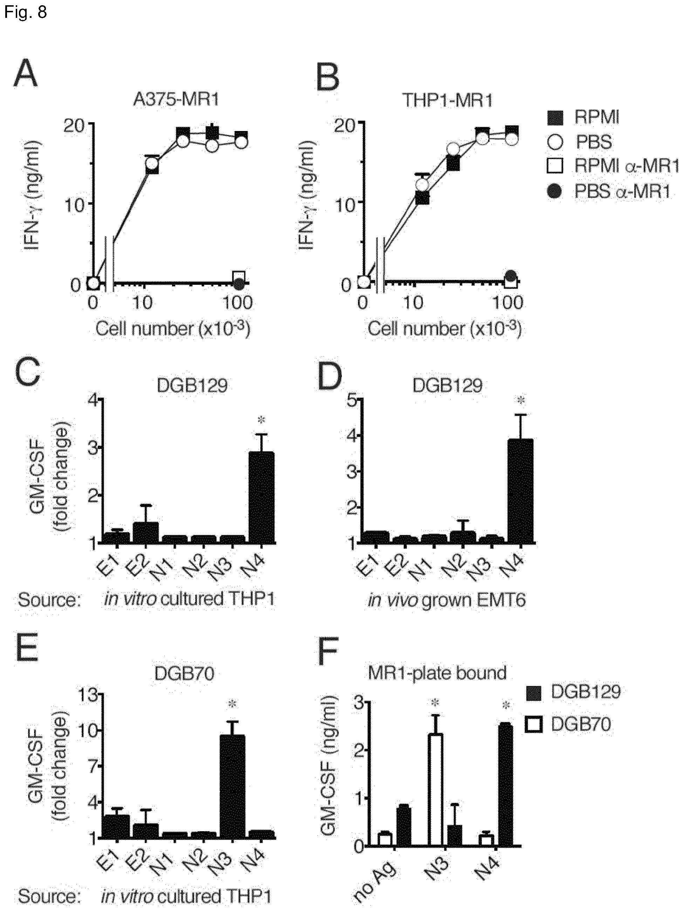

**Figure 8****.** MR1T cells recognize antigens present in tumour cells and not derived from RPMI 1640 medium. Stimulation of the DGB129 MR1T cell clone by MR1-overexpressing (**A**) A375 cells (A375-MR1) and (**B**) THP-1 cells (THP1-MR1) grown for 4 days in RPMI 1640 or in PBS both supplemented with 5% human serum. Inhibition of T cell clone reactivity by anti-MR1 blocking mAbs (α-MR1) is shown. DGB129 cells recognize APCs loaded with fractions isolated from (**C**) THP-1 cell lysate or from (**D**) *in vivo* grown mouse breast tumour EMT6. Fractions E1 and E2 contain hydrophobic molecules; fractions N1-N4 contain hydrophilic molecules. (**E**) DGB70 MR1T cells react to N3 fraction of THP-1 lysate. (**F**) Stimulation of DGB129 and DGB70 T cells by THP-1-derived fractions N3 and N4 loaded onto plastic-bound recombinant MR1. Shown is T cell release of IFN-γ or GM-CSF mean ± SD of duplicate cultures (representative of three independent experiments). Total cytokine release is shown in panels A, B, F; fold increase over background is shown in panels C, D, E. * P<0.05 (Unpaired Student's t-test).

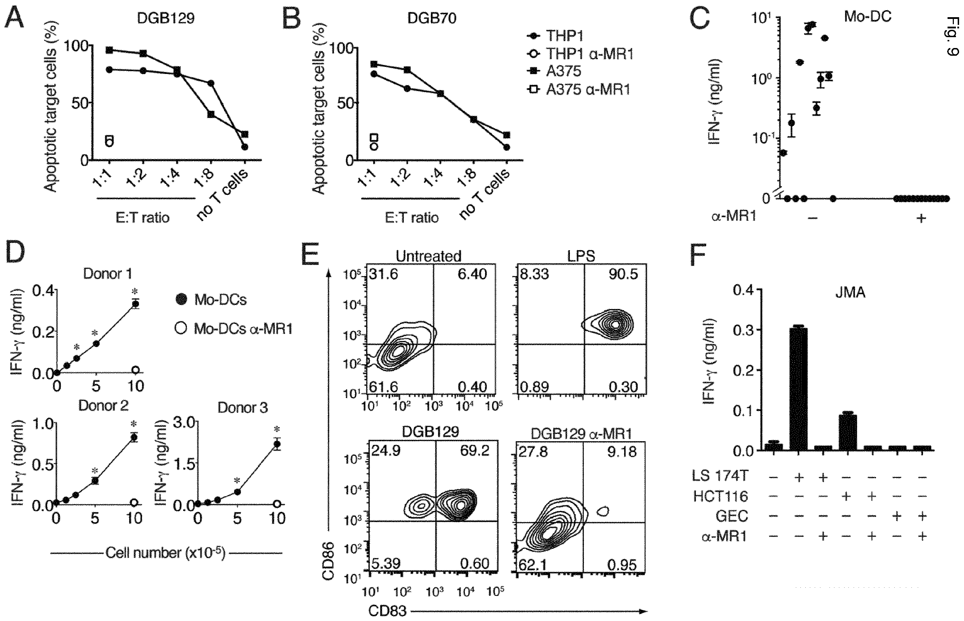

**Figure 9****.** MR1T cells display differential anti-tumour responses. The MR1-expressing tumour cell lines THP-1 and A375 were cultured overnight with the MR1T cell clones (**A**) DGB129 or (**B**) DGB70 at the indicated effector:target (E:T) ratios. The graphs show the percentages of apoptotic target cells in individual experimental conditions, assessed by flow cytometry using Annexin V and propidium iodide staining. MR1T cells were identified by staining with anti-CD3 mAbs and excluded from the analysis. Inhibition of MR1T cell clone killing capacity by anti-MR1 (α-MR1) mAbs is also shown at the 1:1 E:T ratio. (**C**) Recognition of Mo-DCs isolated from a healthy individual by thirteen MR1T cell clones with or without anti-MR1 mAbs (α-MR1). Graphs show IFN-γ release (mean ± SD of duplicate cultures). (**D**) Recognition of Mo-DCs from three donors by the representative DGB129 MR1T cell clone in the absence or presence of anti-MR1 (α-MR1) mAbs. IFN-γ release in the supernatants is shown and expressed as mean ± SD. **(E)** Flow cytometry analysis of co-stimulatory molecules CD83 and CD86 on Mo-DCs after co-culture with DGB129 MR1T cells with or without anti-MR1 mAbs (α-MR1). A control group consisting of Mo-DCs stimulated with LPS (10 ng/ml) in the absence of T cells is also shown. Numbers indicate percentages of cells in each quadrant. (**F**) Stimulation of JMAN MR1T cell clone by LS 174T and HCT116 gastrointestinal tumour cell lines and by normal gut epithelial cells (GEC) in the presence or not of anti-MR1 mAbs (α-MR1). Columns show IFN-γ release (mean ± SD of duplicate cultures). All the results are representative of at least three independent experiments. * P<0.05 (Unpaired Student's t-test).

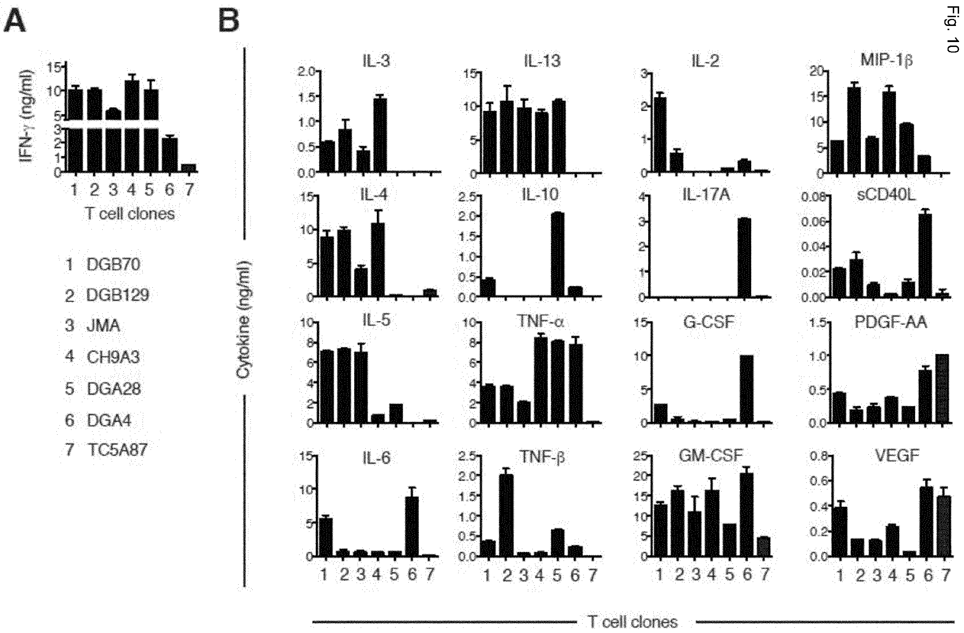

**Figure 10****.** Functional heterogeneity of MR1T cell clones. (**A**) IFN-γ released by 7 selected MR1T cell clones stimulated with A375-MR1 cells. ELISA results are expressed as mean ± SD of IFN-γ release measured in duplicate cultures. (**B**) Analysis of 16 additional cytokines by multiplex cytokine assay performed on the same supernatants for which IFN-γ is shown in **A.** Results are representative of two independent experiments.

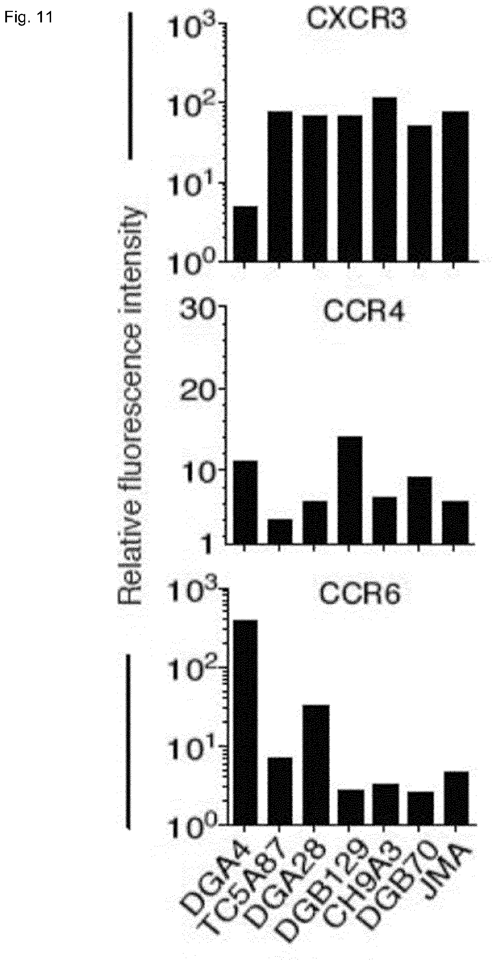

**Figure 11****.** MR1T cell clones display multiple chemokine-receptor expression profiles. Flow cytometry analysis of CXCR3, CCR4 and CCR6 surface expression by seven selected resting MR1T cell clones. Graphs show the relative fluorescence intensity calculated by dividing the median fluorescence intensity (MFI) of specific mAb staining by the MFI of the corresponding isotype control. Data are representative of two independent experiments.

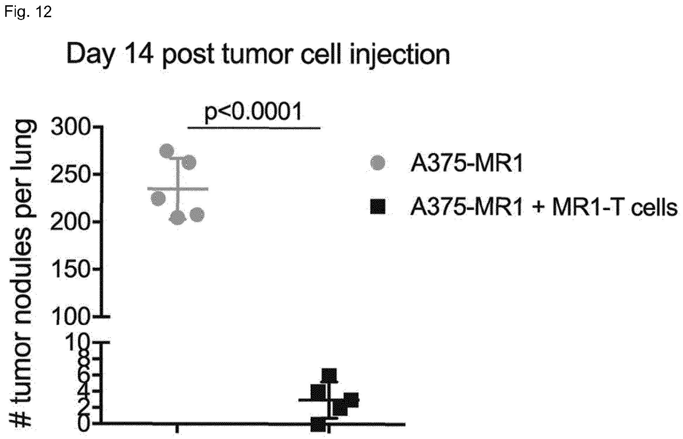

**Figure 12****.** MR1T cells reduce the number of human melanoma lung nodules in mice. Immunocompromised NSG mice were injected with the human melanoma A375 cells expressing MR1 (A375-MR1) and with MR1T cells. On day 14, mice were sacrificed and lung nodules were counted after India ink perfusion. P<0.0001 (Unpaired Student's t-test).

**Table 1. Phenotype of select MR1-reactive T cell clones.**

**Table 2. List of tumour cell lines recognized by MR1T cells.**

**Table 3. List of TCR protein sequences.**

**Table 4. List of TCR nucleotide sequences.**

### Examples

### Methods

Cells. The following human cell lines were obtained from American Type Culture Collection: A375 (melanoma), THP-1 (myelomonocytic leukemia), J.RT3-T3.5 (TCRβ-deficient T cell leukemia), LS 174T (colon adenocarcinoma), HCT116 (colon carcinoma), Huh7 (hepatocellular carcinoma), HEK 293 (human embryonic kidney), and CCRF-SB (acute B cell lymphoblastic leukemia). SKW-3 cells (human T cell leukemia deficient in TCRα, β, γ and δ genes) were obtained from the Leibniz-Institute DSMZ-German Collection of Microorganisms and Cell Cultures. Two representative MAlT clones (MRC25 and SMC3) and one TCR γδ clone, (G2B9) (Gober et al., The Journal of experimental medicine 197, 163-168 (2003)) were used in this study as control cells and were generated from blood of two healthy donors and maintained in culture as previously described (Lepore et al., Nat Commun 5, 3866 (2014)). MR1T cells were isolated from the peripheral blood of healthy individuals after informed consent was obtained from donors at the time of blood collection under approval of the "Ethikkommision Nordwest und Zentralschweiz/EKNZ (139/13). Briefly, T cells purified by negative selection (EasySep^{™} Human T Cell Enrichment Kit, StemCell) were stimulated with irradiated (80 Gray) A375-MR1 cells (ratio 2:1) once a week for three weeks. Human rIL-2 (5 U/ml; Hoffmann-La Roche), rIL-7 and rIL-15 (both at 5 ng/ml, Peprotech) were added at day +2 and +5 after each stimulation. Twelve days after the last stimulation cells were washed and co-cultured overnight with A375-MR1 cells (ratio 2:1). CD3⁺CD69⁺CD37⁺ cells were then sorted and cloned by limiting dilution in the presence of PHA (1 µg/ml, Wellcome Research Laboratories), human rIL-2 (100 U/ml, Hoffmann-La Roche) and irradiated PBMC (5×10⁵ cells /ml). In other experiments, MR1T cells clones were generated using the same protocol from sorted CD3⁺CD69⁺CD137⁺ upon a single overnight stimulation with A375-MR1 cells (ratio 2:1). T cell clones were periodically re-stimulated following the same protocol (Lepore et al., ibid.). Monocytes and B cells were purified (>90% purity) from PBMCs of healthy donors using EasySep Human CD14 and CD19 positive selection kits (Stemcell Technologies) according to the manufacturer instructions. Mo-DCs were differentiated from purified CD14⁺monocytes by culture in the presence of GM-CSF and IL-4 as previously described (Lepore et al., ibid.). Human normal gut epithelial cells (GEC) were isolated from gut biopsies of tumour-free individuals according to a published protocol (Graves et al., Journal of immunological methods 414, 20-31 (2014)).

Generation of cells expressing MR1A gene covalently linked with β2m. A human MR1A cDNA construct linked to β2m via a flexible Gly-Ser linker was generated by PCR as previously described (Lepore et al., ibid.). The K43A substitution in the MR1A cDNA was introduced into the fusion construct using the following primers: MR1K43A_f 5'-CTCGGCAGGCCGAGCCACGGGC (SEQ ID 53) and MR1K43A_r 5'GCCCGTGGCTCGGCCTGCCGAG (SEQ ID 54). Resulting WT and mutant constructs were cloned into a bidirectional lentiviral vector (LV) (Lepore et al., ibid.). HEK 293 cells were transfected with individual LV-MR1A-β2m constructs together with the lentivirus packaging plasmids pMD2.G, pMDLg/pRRE and pRSV-REV (Addgene) using Metafectene Pro (Biontex) according to manufacturer instructions. A375, and THP-1, cells were transduced by spin-infection with virus particle containing supernatant in the presence of 8 µg/ml protamine sulfate. Surface expression of MR1 was assessed by flow cytometry and positive cells were FACS sorted.

Soluble recombinant β2m-MR1-Fc fusion protein. β2m-MR1-Fc fusion construct was obtained using human MR1A-β2m construct described above as template. DNA complementary to β2m-MR1A gene was amplified by PCR using primers: β2mXhol_f 5'-CTCGAGATGTCTCGCTCCGTGGCCTTA (SEQ ID 55) and MR1-lgG1_r 5'-GTGTGAGTTTTGTCGCTAGCCTGGGGGACCTG (SEQ ID 56), thus excluding MR1 transmembrane and intracellular domains. The DNA complementary to the hinge region and CH2-CH3 domains of human IgG1 heavy chain was generated using the following primers: Nhel-hinge-f 5'-CAGGTCCCCCAGGCTAGCGACAAAACTCACAC (SEQ ID 57) and IgG1Notl_r 5'-GCGGCCGCTCATTTACCCGGAGACAGGGAGA (SEQ ID 58) from pFUSE-hlgG1-Fc1 (InvivoGen). The β2m-MR1A and IgG1 PCR products were joined together using two-step splicing with overlap extension PCR and the resulting construct subcloned into the Xhol/Notl sites of the BCMGSNeo expression vector. CHO-K1 cells were transfected with the final construct using Metafectene Pro (Biontex), cloned by limiting dilutions and screened by ELISA for the production of β2m-MR1-Fc fusion protein. Selected clones, adapted to EX-CELL ACF CHO serum-free medium (Sigma), were used for protein production and β2m-MR1-Fcwas purified using Protein-A-Sepharose (Thermo Fisher Scientific) according to manufacturer instructions. Protein integrity and purity were verified by SDS-PAGE and Western Blot using anti-MR1 mAb 25.6 (Biolegend).

Flow cytometry and antibodies. Cell surface labeling was performed using standard protocols. Intracellular labeling was performed using the True-Nuclear^{™} Transcription Factor Buffer Set according to the manufacturers' instructions. The following anti-human mAbs were obtained from Biolegend: CD4-APC (OKT4), CD8α-PE (TuGh4), CD161-Alexa Fluor 647 (HP-3G10), CD69-PE (FN50), CD3-PE/Cy7, Brilliant Violet-711, or Alexa-700 (UCHT1), CD137-biotin (n4b4-1), CXCR3-BrilliantViolet 421 (G025H7), CD83-biotin (HB15e), MR1-PE (26.5) and TRAV1-2-PE (10C3). CD86-FITC (2331), CCR4-PECy7 (1G1) and CCR6-PE (11A9) mAbs were from BD Pharmingen. All these mAbs were used at 5 µg/ml. Biotinylated mAbs were revealed with streptavidin-PE, -Alexa Fluor 488, or -Brilliant violet 421 (2 µg/ml, Biolegend). Samples were acquired on LSR Fortessa flow cytometer (Becton Dickinson). Cell sorting experiments were performed using an Influx instrument (Becton Dickinson). Dead cells and doublets were excluded on the basis of forward scatter area and width, side scatter, and DAPI staining. All data were analyzed using FlowJo software (TreeStar).

TCR gene analysis of MR1T cell clones. TCRα and β or gene TCRγ and δ expression by MR1T cell clones was assessed either by RT-PCR using total cDNA and specific primers, or by flow cytometry using the IOTest^{®} Beta Mark TCR Vβ Repertoire Kit (Beckman Coulter) according to the manufacturers' instructions or panγδ TCR-specific monoclonal antibodies (B1, Biolegend). For RT-PCR, RNA was prepared using the NucleoSpin RNA II Kit (Macherey Nagel) and cDNA was synthesized using Superscript III reverse transcriptase (Invitrogen). TCRα, β, γ and δ cDNAs were amplified using sets of Vα, Vβ, Vγ and Vδ primers as directed by the manufacturer (TCR typing amplimer kit, Clontech). Functional transcripts were identified by sequencing and then analyzed using the ImMunoGeneTics information system (http://www.imgt.org).

TCR gene transfer. TCRα and β functional cDNA from the MAlT cell clone MRC25 were cloned into the *Xhol*/*Notl* sites of the BCMGSNeo expression vector (Karasuyama and Melchers Eur. J. Immunol. 1988 18:97-104) and the resulting constructs were used to co-transfect J.RT3-T3.5 cells by electroporation according to standard procedure. Transfectants expressing TRAV1-2 and CD3 were FACS sorted. The TCRα and β or TCRγ and δ functional cDNAs from MR1T clones were cloned into the *Xma*I/*BamHI*sites of a modified version of the plasmid 52962 (Addgene) expression vector. SKW-3 cells were transduced with virus particle-containing supernatant generated as described above. Cells were FACS sorted based on CD3 expression.

Fractionation of cell and whole tumour lysates. Total cell lysates were generated from a single pellet of 2.5×10⁹ THP-1 cells via disruption in water with mild sonication. The sonicated material was then centrifuged (15,000g for 15 min at 4°C) and the supernatant collected (S1). Next, the pellet was re-suspended in methanol, sonicated, centrifuged as before, and the supernatant obtained was pooled with the S1 supernatant. The final concentration of methanol was 10%. The total cell extract was then loaded onto a C18 Sep-Pak cartridge (Waters Corporation) and the unbound material was collected and dried (fraction E-FT). Bound material was eluted in batch with 75% (fraction E1) and 100% methanol (fraction E2). The E-FT material was re-suspended in acetonitrile/water (9:1 vol/vol) and loaded onto a NH₂ Sep-Pak cartridge (Waters Corporation). Unbound material (fraction N-FT) and 4 additional fractions were eluted with increasing quantities of water. Fraction N1 was eluted with 35% H₂O, fraction N2 with 60% H₂O, fraction N3 with 100% H₂O, and fraction N4 with 100% H₂O and 50 mM ammonium acetate (pH 7.0). All fractions were dried and then re-suspended in 20% methanol (fractions E1, E2 and N-FT) or 100% H₂O (all other fractions) prior to being stored at -70°C.

Mouse EMT6 breast tumours were prepared as described (Zippelius et al., Cancer Immunol Res 3, 236-244 (2015)). Freshly excised tumours were extensively washed in saline, weighted and 4 g masses were homogenized in 7 ml of HPLC-grade water using a Dounce tissue grinder. Tumour homogenate underwent two freeze-thaw cycles, centrifuged (3,250g) for 10 min at 4°C, and supernatant was collected and stored at -70°C. The pellet was extracted a second time with 2 ml of HPLC-grade water, centrifuged (5,100g) for 10 min at 4°C and the supernatant was collected and stored at -70°C. The pellet was further extracted with 9 ml of HPLC-grade methanol for 5 min at room temperature by vortexing, centrifuged (5,100g) for 10 min at 4°C, and supernatant collected. The three supernatants were pooled, dried, and resuspended in water:methanol (10:1). Material was fractionated using C18 and NH₂ Sep-Pak cartridges as above.

T cell activation assays. MR1-restricted T cells (5×10⁴/well unless otherwise indicated) were co-cultured with indicated target cells (5×10⁴/well) in 200 µl total volume in duplicates or triplicates. T cells were cultured with indicated APCs for 24 h. In some experiments, anti-MR1 mAbs (clone 26.5) or mouse IgG2a isotype control mAbs (both at 30 µg/ml) were added and incubated for 30 min prior to the addition of T cells. *E. coli* lysate was prepared from the DH5α strain (Invitrogen) grown in LB medium and collected during exponential growth. Bacterial cells were washed twice in PBS and then lysed by sonication. After centrifugation (15,000g for 15 min), the supernatant was collected, dried, and stored at -70°C. APCs were pulsed for 4 h with *E. coli* lysate equivalent to 10⁸ CFU/ml (unless otherwise indicated) before addition of T cells. In some experiments, APCs were pre-incubated with 6-FP or Ac-6-FP (Schircks Laboratories) for 4 h before co-culture with T cells. In control experiments with TCR γδ cells expressing TCR Vγ9 and Vδ2 chains, the APCs were first treated for 6 h with zoledronate (10 µg/ml) prior to T cell addition. Activation experiments with plate-bound recombinant human β2m-MR1-Fc were performed by coating β2m-MR1-Fc onto 96 well plates (4 µg/ml) and loading with cartridge-purified cell lysates for 4 h at 37°C before washing twice and adding T cells. Supernatants were collected after 24 h and IFN-γ or GM-CSF were assessed by ELISA. Multiple cytokines and chemokines in cell culture supernatants were analyzed using the Milliplex MAP human cytokine/chemokine magnetic bead panel - Premixed 41 plex (HCYTMAG-60K-PX41; Merck Millipore) according to the manufacturer's instructions. Samples were acquired on a Flexmap 3D system (Merck Millipore) and Milliplex analyst software was used to determine mean fluorescence intensity and analyte concentration.

Killing of tumour cells. Killing assays were performed using target cell lines (2×10⁴ cells/ml) incubated either alone or with T cells at different E/T ratios for 24 h, in the presence or absence of anti-MR1 mAb (30 µg/ml, clone 26.5). The target cells were stained with PE-Annexin V (BD) and propidium iodide (PI) (Sigma-Aldrich), as previously described (2). T cells were identified by staining with anti-CD3 mAbs and excluded from the analysis. Apoptosis was evaluated as follows: Annexin V⁺ PI⁺ = advanced apoptosis and Annexin V⁻ PI⁺ = necrosis. The percentage of apoptotic + necrotic cells in the absence of T cells (spontaneous apoptosis; no T cells) is also shown.

Statistics. Data were analyzed using Unpaired Student's t-test (Prism 6, GraphPad software).

Identification and characterization of novel tumour-reactive MR1-restricted T cells in healthy donors

The inventors detected an atypical MR1-restricted T cell clone that did not react to microbial ligands during earlier studies on the repertoire of human MAIT cells. This T cell clone (DGB129) recognized cell lines constitutively displaying surface MR1 (CCRF-SB lymphoblastic leukemia cells, or THP-1 monocytic leukemia cells; **Figure 1A**) or transfected with the MR1 gene (A375 melanoma cells; A375-MR1; **Figure 1A**) in the absence of any exogenously added antigens (**Figure 1B**). Sterile recognition of MR1⁺ target cells was fully inhibited by blocking with anti-MR1 monoclonal antibodies (mAbs) **(****Figure 1B****),** and thus resembled the MAlT cell response to *E. coli-*derived antigens assessed in parallel **(****Figure 1C****).** Importantly, DGB129 T cells also failed to recognize the synthetic MAlT cell agonist 6,7-dimethyl-8-D-ribityllumazine (RL-6,7-diMe; **Figure 1D****),** differently from a control MAlT cell clone, which instead was stimulated in MR1-dependent manner by this compound **(****Figure 1E****).** DGB129 cells did not express the canonical semi-invariant TCR typical of MAlT cells **(Table** 1).

The inventors asked whether the DGB129 clone was representative of a novel population of tumour-reactive MR1-restricted T cells different from microbe-reactive MAlT cells. They therefore established a method to isolate and study these unpredicted MR1-restricted T cells. Purified T cells from two healthy donors were labelled with the proliferation marker CellTrace violet (CTV) and stimulated with irradiated A375-MR1 cells in the absence of exogenous antigens. Proliferating cells were re-challenged with A375-MR1 cells and those expressing high levels of the activation marker CD137 were sorted and cloned by limiting dilution (**Figure 2A**). Individual T cell clones were then interrogated for their capacity to recognize A375-MR1 and A375 cells lacking MR1 (A375-WT). In both donors the inventors found that a major fraction of T cell clones (126/195 and 37/57, respectively) displayed specific recognition of A375-MR1 cells (**Figure 2B****,D**), which was inhibited byanti-MR1 blocking mAbs (**Figure 2C****,E**). Staining with TCR Vβ-specific mAbs of 12 MR1-reactive T cell clones revealed that they expressed 7 different TRBV chains (TRBV4-3, 6-5/6-6/6-9, 9, 18, 25-1, 28, 29-1) with some of the clones sharing the same TRBV gene. Furthermore, none expressed the TRAV1-2 chain, canonical for MAlT cells.

Lack of specific markers did not allow univocal identification of these novel T cells ex *vivo* by standard flow cytometry. Therefore, their frequency was estimated by combining flow cytometry analysis after very short-time *in vitro* stimulation and single T cell cloning experiments. Purified blood T cells from five healthy donors were co-cultured overnight with MR1-deficient or MR1-sufficient A375 cells and analysed for the expression of the activation markers CD69 and CD137 (**Figure 3A**). In all of the five donors screened, the percentage of CD69⁺CD137⁺ T cells detected was consistently higher after stimulation with A375-MR1 cells (range 0.034-0.072% of T cells) than after co-culture with A375-WT cells (range 0.015-0.032%) (**Figure 3A****,B**). As the two types of APCs differed for MR1 expression, MR1-reactive T cells accounted for the increased numbers of activated T cells after stimulation with MR1-positive APCs. Using this approach, the inventors estimated that the circulating T cell pool of the analysed individuals contained A375-MR1-reactive T cells at frequency ranging between 1:2,500 (0.072-0.032=0.04%) and 1:5,000 (0.034-0.015=0.019%). This estimated frequency is higher than the frequency of peptide-specific CD4⁺ T cells after antigen exposure (Lucas et al., J Virol 78:7284-7287; Su et al., Immunity 38:373-383). These observations were supported by parallel experiments in which sorted CD69⁺CD137⁺ overnight-activated T cells from one of these donors (Donor C, **Figure 3A****,** right panel) were cloned. Indeed, 31 out of 96 screened T cell clones (32%) displayed specific reactivity to A375-MR1 cells (**Figure 3C**), which was inhibited by anti-MR1 mAbs (**Figure 3D**). Accordingly, the calculated frequency of A375-MR1-responsive T cells among blood T cells of this donor was 1:5,000 (0.065x0.32= 0.02%), a value consistent with the estimated range. Detailed analysis of representative T cell clones derived from three donors confirmed that they displayed diverse TCRα and β chains and indicated differential expression of CD4, CD8 and CD161 (**Table 1**).

Collectively, these findings suggested that the identified tumour-reactive MR1-restricted T cells are a novel yet common polyclonal population of lymphocytes in the blood of healthy human individuals (hereafter termed MR1T cells).

### MR1T cell TCR gene transfer confers MR1-restricted recognition of tumour cells

The inventors next investigated whether MR1T cell reactivity to tumour cells was mediated by the TCR. Expression of paired TCRα and β genes cloned from different MR1T cell clones in the TCR-deficient SKW-3 cells, conferred MR1 recognition of tumour cells which was comparable to that displayed by the original MR1T cells and which was completely blocked by anti-MR1-mAbs (**Figure 4A-C**). In control experiments, transfer of TCRα and β genes of a representative MAlT cell clone conferred the ability to recognize A375-MR1 cells in MR1-dependent manner only in the presence of *E. coli* antigens (**Figure 4D**). These data highlighted the critical role of the TCR in mediating MR1T cell recognition of tumour cells and suggested that MR1T cell TCR gene transfer can effectively redirect the reactivity of selected T cells toward MR1-expressing tumour cells.

### Differential recognition of tumour cells by MR1T cell clones

Having generated a large panel of MR1T cell clones reacting to MR1-expressing A375 melanoma cells, the inventors next investigated whether they could also recognize other types of tumour cells constitutively expressing surface MR1, including THP-1 myelomonocytic cells, Huh7 hepatoma cells, HCT116 colon carcinoma cells and LS 174T goblet-like colon adenocarcinoma cells. All these cell types supported MAlT cell activation in the presence of microbial antigens and in an MR1-dependent manner (**Figure 5A**)**.** The same cells were able to induce sterile activation of select MR1T cell clones to various extents. THP-1 cells were recognized by the majority of the tested MR1T cell clones, followed by the Huh7 hepatoma cells, the LS 174T goblet-like cells and the HCT116 colon carcinoma cells (**Figure 5B**). Importantly, all responses were blocked by anti-MR1 mAbs.

These data further confirmed that MR1T cells are a novel and heterogeneous population of tumour-reactive T cells restricted to the non-polymorphic antigen-presenting molecule MR1.

### MR1T cells recognize MR1-bound antigens present in tumour cells

The inventors next studied the basis of MR1T cell reactivity to tumour cells. First, they sought to definitively rule out the possibility that MR1T cell clones could recognize microbial antigens, in analogy to MAlT cells. While a control MAIT cell clone reacted to A375-MR1 cells only in the presence of *E. coli* lysate, activation of different MR1T cell clones was not enhanced by the *E. coli* lysate (**Figure 6A**). Consistent with these data, MR1-negative A375-WT cells failed to stimulate either type of T cells, irrespective of whether *E. coli* lysate was added, (**Figure 6A**) and importantly anti-MR1 mAbs efficiently blocked both MR1T and MAlT cell responses (**Figure 6A**). These findings confirmed that microbial ligands present in *E. coli* and stimulating MAlT cells do not stimulate the tested MR1T cells.

The inventors then tested the response of MR1T cells to the known MR1 ligands 6-FP and Ac-6-FP, which have previously been reported to stimulate a rare subset of TRAV1-2-negative T cells and inhibit MAlT cell activation by microbial antigens. MR1T cell stimulation was impaired in the presence of 6-FP or Ac-6-FP ligands, which also impaired *E. coli* stimulation of control MAIT cells, but did not disrupt control TCR γδ cell responses to cognate antigen presented by the same APCs, thus excluding compound toxicity (**Figure 6B****,C and 7A-C**)**.** Notably, 6-FP or Ac-6-FP failed to inhibit the activation of MR1T cells or MAlT cells when the target A375 cells were transduced to express mutant MR1 molecules with defective ligand binding capacity (blockade of Schiff base formation with ligands by mutation of Lysine 43 into Alanine, A375-MR1 K34A; **Figure 6B****,C and 7D,E**). The specific inhibition observed with 6-FP or Ac-6-FP indicated that MR1T cells i) do not recognize 6-FP and Ac-6-FP, ii) react to MR1-bound cellular antigens, and iii) are stimulated by ligands that do not require the formation of a Schiff base with MR1.

To gain further information on the origin of the recognized antigens the inventors asked whether the stimulatory capacity of tumour target cells was dependent on culture medium constituents, as some MR1 ligands, *e.g.* 6-FP, may derive from folate present in RPMI 1640 medium used for cell culture. Both THP-1 and A375-MR1 cells were extensively washed and cultivated 4 days in phosphate buffered saline solution (PBS) supplemented exclusively with 5% human serum. Cells were washed daily before being used to stimulate DGB129 MR1T cells and the T cell activation assays were performed in PBS. THP-1 and A375-MR1 cells grown in RPMI 1640 or in PBS showed the same stimulatory capacity (**Figure 8A****,B**), thus indicating that medium constituents are not responsible for MR1T cell activation. To directly investigate whether the stimulatory antigens were present in target tumour cells, the inventors then performed T cell activation assays using as source of antigen two types of tumour lysates. The first lysate was obtained from *in vitro* cultured THP-1 cells, while the second one was prepared from mouse breast tumours immediately after resection. Two hydrophobic and four hydrophilic fractions were obtained and tested using as APCs THP-1 cells that constitutively express low levels of MR1. The DGB129 clone reacted only to fraction N4, containing highly hydrophilic compounds isolated from both freshly explanted mouse tumour and *in vitro* cultured THP-1 cells (**Figure 8C****,D**). These results ruled out the possibility that stimulatory antigens were derived from RPMI 1640 components and indicated their cellular origin. The inventors also tested the fractions generated from THP-1 lysates with DGB70, another representative MR1T cell clone. DGB70 cells recognized fraction N3 and not N4, (**Figure 8E**), suggesting that at least two distinct compounds differentially stimulated the two MR1T clones. The same fractions were also loaded onto plastic-bound MR1 molecules and showed alternative and specific stimulatory capacity, *i.e.* N3 stimulated only DGB70 cells, while N4 stimulated only DGB129 cells (**Figure 8F**). In the absence of N3 and N4 fractions, the two clones did not react to MR1, further indicating the requirement of specific antigens.

In conclusion, these data indicated that MR1T cells recognize MR1 complexed with ligands not derived from culture medium and present also in tumour cells grown *in vivo.*

### MR1T cells display differential anti-tumour responses

To assess the anti-tumour activity of MR1T cells the inventors tested their capacity to directly kill tumour cells *in vitro.* Two representative MR1T cell clones (DGB129 and DGB70) efficiently killed both MR1-expressing THP-1 and A375 cells at various effector:target ratios (**Figure 9A,B****).** A control MAIT cell clone failed to kill these two cell types, although it was fully capable of killing when targets were *E*. *coli*-infected (**not shown**). These results indicated that MR1T cells display specific cytotoxic activity against MR1-expressing tumour cells.

Having found that MR1 T cells recognized and killed the myelomonocytic tumour cell line THP-1, the inventors next addressed whether they could also recognize normal myeloid cells including monocytes and monocyte-derived dendritic cells (Mo-DC) from different donors. Monocytes were not recognized by any of the tested MR1T cell clones (**not shown**). By contrast, some MR1T cell clones reacted to Mo-DC in MR1 dependent manner (**Figure 9C**). Interestingly, experiments performed with the representative DGB129 MR1T cell clone revealed that recognition of Mo-DC did not result in Mo-DC killing (**not shown**), but promoted up-regulation of CD83 and CD86 activation markers by Mo-DC (**Figure 9D**). Remarkably, the activation of Mo-DC induced by DGB129 cells was fully inhibited by anti-MR1 mAbs (**Figure 9D**). These data suggested that some tumour-reactive MR1T cells elicit direct anti-tumour activity and also promote activation of innate immune cells, with important implications in the establishment of effective anti-tumour immune responses.

As the inventors observed that some MR1T cell clones reacted to HCT116 and LS 174T intestinal tumour cells, they next investigated whether they could also recognize normal gut epithelial cells (GEC) prepared from gut biopsies. GEC cells were not stimulatory for any of the tested HCT116- or LS 174T-reactive MR1T cell clones (**Figure 9F****,G**), thus suggesting that MR1T cell clones may display specific recognition of gastrointestinal tumour cells while not reacting to normal intestinal epithelial cells.

To further assess the specificity of tumour recognition by MR1T cells, the inventors finally investigated whether they could react to other types of normal cells including neutrophils, NK cells, B cells and T cells. None of these cells were recognized by the tested MR1T cells (**not shown**).

Collectively, these data identify MR1T cells as a novel and heterogeneous population of human MR1-restricted T lymphocytes that i) differently react to various types of tumour cells, ii) display cytotoxic activity against tumour cells, iii) do not recognize normal cells with exception of *in* vitro-differentiated Mo-DC, and iv) do not kill Mo-DC but instead induce their activation. These findings suggested that MR1T cells display important anti-tumour properties and deserve to be exploited for their immunotherapeutic potential.

### MR1T cells are functionally heterogeneous

The inventors finally analyzed the cytokine secretion profile of representative MR1T cell clones upon stimulation by A375-MR1 tumour cells. All clones tested released IFN-γ (**Figure 10A**). However, the inventors also observed diverse expression profiles of Th1 (IL-2, TNF-α and TNF-β), Th2 (IL-3, IL-4, IL-5, IL-6, IL-10, IL-13) and Th17 cytokines (IL-17A, G-CSF, GM-CSF), and other soluble factors (MIP-1β, soluble CD40L PDGF-AA and VEGF; **Figure 10B**). The variable combinations and quantities of cytokines expressed by MR1T cells suggested considerable functional plasticity within this population. For example, clone DGA4 secreted large quantities of IL-17A, IL-6, TNF-α and GM-CSF, but failed to secrete the prototypic Th2 cytokines IL-4, IL-5, IL-10 or IL-13, and thus displayed an 'atypical' Th17-like phenotype. In contrast, clone TC5A87 released substantial amounts of VEGF and PGDF-AA, but only little Th1 or Th2 cytokines, and no IL-17A. Notably, four of the seven clones studied (DGB129, CH9A3, DGB70, JMA) displayed a Th2-skewed profile of cytokine release, a functional phenotype which has been recently associated with protective anti-tumour immunity.

The inventors next investigated the expression of three selected chemokine receptors known to be differentially expressed by T cell subsets with distinct functions and whose alternative combined expression regulates T cell recirculation and migration to diverse homing sites. All MR1T cell clones but DGA4 displayed high levels of CXCR3 (**Figure 11**). In addition, the inventors observed divergent expression patterns of CCR4 and CCR6 (**Figure 11**), which further suggested that MR1T cells are heterogeneous.

In a final series of studies it was investigated whether MR1T cells maintain their tumour-killing capacity *in vivo* using a lung solid tumour model. Mice intravenously injected with A375 melanoma cells expressing MR1 received DGB129 cells or were left untreated. On day 14, mice were sacrificed and the number of tumour nodules in the lungs was counted. While untreated mice showed 200-250 nodules, those treated with MR1T cells showed 1-6 nodules (**Figure 12**). These results confirmed that *in vivo* growing tumour cells produce the antigens stimulating MR1T cells. Importantly, they provided strong evidence of the efficient capacity of MR1T cells to kill solid tumour cells *in vivo.*

Taken together, these data indicated that the tumour MR1-reactive T clones tested here are phenotypically and functionally diverse, thus suggesting that MR1T cells include multiple subsets with distinct recirculation patterns and tissue homing capacity and likely different roles in tumour immunity. In conclusion, these data identify MR1T cells as a novel population of human T lymphocytes that recognize MR1 :tumour-associated-antigen complexes and may participate in anti-tumour immune responses with multiple effector functions.

**Table 1. Phenotype of select MR1-reactive T cell clones.**

| Clone | CD4 | CD8α | CD161 | TCRβ |
|---|---|---|---|---|
| DGB129 | - | + | - | TRBV12-4 |
| DGB70 | - | - | - | TRBV28 |
| DGA28 | - | + | + | TRBV29-1 |
| DGA4 | - | - | + | TRBV6-1 |
| JMA | - | + | - | TRBV25-1 |
| TC5A87 | - | + | - | TRBV25-1 |
| CH9A3 | - | + | - | TRBV5-5 |

**Table 2. Tumour cell lines recognized by human MR1T cells.**

| Cell line | Origin |
|---|---|
| A375 | Human melanoma |
| CCRF-SB | Human B lymphoblastic leukemia |
| Huh7 | Human hepatocellular carcinoma |
| HCT116 | Human colon carcinoma |
| LS 174T | Human colon adenocarcinoma |
| THP-1 | Human myelomonocytic leukemia |

### The following examples further illustrate the clinical workflow in which the invention is applied:

### Screening of MR1-expressing cancers

A cancer patient's tissue fresh or fresh-frozen tissue biopsies are analyzed for MR1 expression using mAbs specific for human MR1 and PCR amplification of MR1 mRNA.

Cancer therapy. Example 1: Selection of best MRT1 TCR genes for recognition of primary MR1-expressing cancer cells.
- Primary MR1⁺ cancer cells isolated *ex vivo* are used to stimulate a library of previously characterized MR1T cell clones. Each clone expresses different TCR genes and recognizes different types of cancer cells.
- The MR1T cells clones best responding to the cancer cells of the patient are selected and their TCR genes are used for TCR gene therapy. Response is assayed as a function of cytokine release and / or surface marker expression. Cells are assayed by internal (cytokine) or surface marker staining with antibodies reactive to the assayed activation markers, exemplified but not restricted to CD3, CD69, CD137, CD150, and / or ICOS (surface markers) and INF-γ and GM-CSF (cytokine).
- When available soluble MR1T TCR will be multimerized and used to stain tumor cells isolated from tumour biopsies. The MR1T TCR multimers binding to tumour cells will allow rapid selection of MR1T TCRs suitable for gene therapy in that patient.
- Several circulating patient T cell populations may be used as recipient T cells (naive, central memory, effector memory, CD4⁺, CD8⁺, or CD4, CD8 double negative T cells). Naive T cells are selected to allow unprimed T lymphocytes to mature in the presence of tumor cells when they are transduced with TCR genes recognizing MR1-tumor antigens. Central and effector memory cells are used because they provide immediate proliferation and effector functions (tumor killing) upon recognition of tumor cells expressing MR1. CD4 cells are selected to provide sufficient numbers of T helper cells that facilitate recruitment and expansion of other cells with anti-tumor functions. CD8 T cells are selected to facilitate killing of tumor cells. CD4-CD8 double negative T cells are selected for their innate-like functions such as immediate release of large amounts of killer effector molecules (TNFα, granzymes and granulysin).
- T cells expressing the transduced TCR genes and with selected effector functions are used for adoptive cell therapy (ACT).

T cells from peripheral blood of patients are stained with monoclonal antibodies specific for surface markers (CD4, CD8, CD27, CD45RA, CD57) and sorted. Each sorted population is activated with Dynabeads^{®} Human T-Activator CD3/CD28 (ThermoFisher) and 24 h later transfected with the TCR genes encoding the MR1T TCR selected for the individual patient. This yields a modified T cell preparation (recipient T cells). In some cases, recipient T cells are also modified by gene-editing methods to inactivate PD1, ILT2 and ILT4 inhibitory genes or were transduced with CD137 and CD134 genes to promote cell survival, cell expansion and to enhance anti-cancer effector function.

Lymphodepletion is made in recipient cancer patients using a non-myeloablative chemotherapy preparative regimen (60 mg/kg cyclophosphamide for 2 days and 25 mg/m² fludarabine administered for 5 days) followed by transfer of T cells and IL-2 given at 720,000 IU/kg to tolerance. In some instances, 200 or 1200 centigray (cGy; 1 Gy = 100 rads) total-body irradiation is added to the preparative regimen. T cells expressing the MR1T exogenous TCR genes (the modified T cell preparation) are transferred into recipient.

TCR genes are cloned in safe recombinant lentivirus vectors (see for example Provasi et al., Nat Med 18, 807-815 (2012)), which contain suicide genes and cannot produce mature viral particles in the absence of other helper viruses. In some cases, TCR genes are cloned in vectors containing suicide genes (for examples, see Greco et al., Front Pharmacol 6, 95 (2015)), thus reducing the risks derived from unwanted gene insertion. In some cases RNA encoding the TCR MR1T genes is transfected in recipient cells (see for example Zhao et al. Molecular therapy 13, 151, 2006)).

Cancer therapy. Example 2: Isolation of MR1T cells from tumor-infiltrating lymphocytes (TILs) of patient to be treated.
- Autologous TILs are prepared from the cancer tissue biopsies according to our previously established protocol (De Libero, ibid.).
- T cells are expanded *in vitro* for 2-3 weeks using medium supplemented with IL-2, IL-7, and IL-15.
- Expanded T cells are tested for reactivity against autologous MR1⁺ cancer cells. T cells that increase surface expression of activation markers (CD137, CD150, CD69, ICOS) are considered cancer-specific and if they are inhibited by the presence of anti-MR1 monoclonal antibodies, they are considered MR1-dependent.
- Cancer-reactive T cells are sorted according to the expression of one of above activation markers and expanded and used for ACT, as outlined above.

## Claims

1. A recombinant cell comprising an expression vector encoding a T cell receptor (TCR) protein heterodimer comprising
- an alpha (α) TCR amino acid sequence selected from SEQ ID NOs 001 to 012 and a beta (β) TCR amino acid sequence selected from SEQ ID NOs 013 to 024,
wherein said isolated T cell receptor protein comprises a pair of alpha α TCR and beta β TCR amino acid sequences selected from
a. SEQ ID NOs 001 and 023,
b. SEQ ID NOs 002 and 022,
c. SEQ ID NOs 003 and 021,
d. SEQ ID NOs 004 and 020,
e. SEQ ID NOs 005 and 019,
f. SEQ ID NOs 006 and 017,
g. SEQ ID NOs 007 and 018,
h. SEQ ID NOs 008 and 016,
i. SEQ ID NOs 009 and 015,
j. SEQ ID NOs 010 and 014,
k. SEQ ID NOs 011 and 013,
l. SEQ ID NOs 012 and 024, or
- a gamma (γ) TCR amino acid sequence of SEQ ID NO 025 and a delta (δ) TCR amino acid sequence of SEQ ID NO 026.

2. The recombinant cell-according to claim 1, wherein said recombinant cell is a human T cell derived from
a. peripheral blood or
b. a tumour infiltrating lymphocyte.

3. A preparation of recombinant cells as specified in claim 2, for use as a medicament.

4. The preparation of MT1-specific cells as specified in claim 2, for use in an autologous adoptive T cell therapy.

5. The preparation of MT1-specific cells as specified in claim 2, for use in an allogeneic adoptive T cell therapy.

6. The preparation of T cells for use according to any one of claims 3 to 5, wherein the preparation is administered in treatment of a cancer **characterized by** MR1 expression.

7. The preparation of T cells for use according to claim 6, wherein the cancer is melanoma.

8. The preparation of T cells for use according to claim 6, wherein the cancer is hepatocellular carcinoma.

9. The preparation of T cells for use according to claim 6, wherein the cancer is colon carcinoma or colon adenocarcinoma.

10. The preparation of T cells for use according to claim 6, wherein the cancer is leukemia.

11. The preparation of T cells for use according to claims 6, wherein the cancer is myelomonocytic leukemia.

12. The preparation of T cells for use according to claim 7, wherein the pair of α TCR and β TCR amino acid sequences is selected from
a. SEQ ID NOs 001 and 023,
b. SEQ ID NOs 002 and 022,
c. SEQ ID NOs 003 and 021,
d. SEQ ID NOs 004 and 020,
e. SEQ ID NOs 005 and 019,
f. SEQ ID NOs 007 and 018, or
g. SEQ ID NOs 010 and 014.

13. An isolated T cell receptor protein that binds to an MR1 molecule, wherein said isolated T cell receptor protein comprises:
- a pair of α TCR and β TCR amino acid sequences selected from
a. SEQ ID NOs 001 and 023,
b. SEQ ID NOs 002 and 022,
c. SEQ ID NOs 003 and 021,
d. SEQ ID NOs 004 and 020,
e. SEQ ID NOs 005 and 019,
f. SEQ ID NOs 006 and 017,
g. SEQ ID NOs 007 and 018,
h. SEQ ID NOs 008 and 016,
i. SEQ ID NOs 009 and 015,
j. SEQ ID NOs 010 and 014,
k. SEQ ID NOs 011 and 013,
l. SEQ ID NOs 012 and 024, or
- a gamma (γ) TCR amino acid sequence of SEQ ID NO 025 and a delta (δ) TCR amino acid sequence of SEQ ID NO 026.

14. An expression vector comprising, and leading to the transcription of, a nucleic acid sequence encoding a functional T cell receptor heterodimer,
said nucleic acid being controlled by a promoter sequence operable in a mammalian cell, wherein the T cell receptor heterodimer is selected from the pairs of:
a. SEQ ID NOs 001 and 023,
b. SEQ ID NOs 002 and 022,
c. SEQ ID NOs 003 and 021,
d. SEQ ID NOs 004 and 020,
e. SEQ ID NOs 005 and 019,
f. SEQ ID NOs 006 and 017,
g. SEQ ID NOs 007 and 018,
h. SEQ ID NOs 008 and 016,
i. SEQ ID NOs 009 and 015,
j. SEQ ID NOs 010 and 014,
k. SEQ ID NOs 011 and 013,
l. SEQ ID NOs 012 and 024, and
m. SEQ ID NO 025 and 026.
